(19) Europäisches Patentamt
European Patent Office
Office européen des brevets

(11) EP 1 140 905 B1

(12) **EUROPEAN PATENT SPECIFICATION**

(45) Date of publication and mention of the grant of the patent:
**14.05.2003 Bulletin 2003/20**

(21) Application number: **99967352.8**

(22) Date of filing: **15.12.1999**

(51) Int Cl.⁷: **C07D 401/14**, C07D 401/12, C07D 417/14, C07D 405/14, C07D 213/82, A61K 31/4545, A61K 31/4427, A61P 7/02

(86) International application number:
**PCT/US99/29887**

(87) International publication number:
**WO 00/039117 (06.07.2000 Gazette 2000/27)**

(54) **HETEROAROMATIC AMIDES AS INHIBITOR OF FACTOR XA**

HETEROAROMATISCHE AMIDE ALS INHIBITOREN VON FAKTOR XA

AMIDES HETEROAROMATIQUES COMME INHIBITEURS DU FACTEUR Xa

(84) Designated Contracting States:
**AT BE CH CY DE DK ES FI FR GB GR IE IT LI LU MC NL PT SE**

(30) Priority: **23.12.1998 US 113452 P**

(43) Date of publication of application:
**10.10.2001 Bulletin 2001/41**

(73) Proprietor: **ELI LILLY AND COMPANY**
**Indianapolis, Indiana 46285 (US)**

(72) Inventors:
- **BEIGHT, Douglas, Wade**
  **Frankfort, IN 46041 (US)**
- **CRAFT, Trelia, Joyce**
  **Indianapolis, IN 46236 (US)**
- **FRANCISKOVICH, Jeffry, Bernard**
  **Zionsville,Indiana 46077 (US)**
- **GOODSON, Theodore, Junior**
  **Indianapolis, IN 46226 (US)**
- **HALL, Steven, Edward**
  **Chapel Hill, NC 27514 (US)**
- **HERRON, David, Kent**
  **Indianapolis, IN 46220 (US)**
- **JOSEPH, Sajan**
  **Indianapolis, IN 46228 (US)**
- **KLIMKOWSKI, Valentine, Joseph**
  **Carmel, IN 46033 (US)**
- **MASTERS, John, Joseph**
  **Fishers, IN 46038 (US)**
- **MENDEL, David**
  **Indianapolis, IN 46236 (US)**
- **MILOT, Guy**
  **Chapel Hill,North Carolina 27516 (US)**
- **PINEIRO-NUNEZ, Marta, Maria**
  **Brownsburg, IN 46112 (US)**
- **SAWYER, Jason, Scott**
  **Indianapolis, IN 46220 (US)**
- **SHUMAN, Robert, Theodore**
  **Sedona, AZ 86336 (US)**
- **SMITH, Gerald, Floyd**
  **Greenwood, IN 46143 (US)**
- **TEBBE, Anne, Louise**
  **22391 Hamburg (DE)**
- **TINSLEY, Jennifer, Marie**
  **Ypsilanti,Michigan 48197 (US)**
- **WEIR, Leonard, Crayton**
  **Westfield, Indiana 46074 (US)**
- **WIKEL, James, Howard**
  **Greenwood, IN 46142 (US)**
- **YEE, Ying, Kwong**
  **Carmel, IN 46033 (US)**
- **KYLE,Jeffrey**
  **Fishers, Indiana 46038 (US)**
- **Wiley,Michael**
  **Indiana 46286 (US)**

(74) Representative: **Pritchard, Judith**
**Eli Lilly and Company Limited**
**Lilly Research Centre**
**Erl Wood Manor**
**Windlesham, Surrey GU20 6PH (GB)**

(56) References cited:
**WO-A-99/00128** **US-A- 5 576 343**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European Patent Convention).



Printed by Jouve, 75001 PARIS (FR)

## Description

This application claims the benefit of U.S. Provisional Application No. 60/113,452, filed 23 December 1998.

This invention relates to antithrombotic heteroaromatic amides which demonstrate activity as inhibitors of factor Xa and, accordingly, which are useful anticoagulants in mammals. In particular it relates to heteroaromatic amides having high anticoagulant activity, and antithrombotic activity. Thus, this invention relates to new amides which are inhibitors of factor Xa, pharmaceutical compositions containing the amides as active ingredients, and the use of the amides as anticoagulants for the manufacture of a medicament for prophylaxis and treatment of thromboembolic disorders such as venous thrombosis, pulmonary embolism, arterial thrombosis, in particular myocardial ischemia, myocardial infarction and cerebral thrombosis, general hypercoagulable states and local hypercoagulable states, such as following angioplasty and coronary bypass operations, and generalized tissue injury as it relates to the inflammatory process. In addition, the heteroaromatic amides are useful as anticoagulants in *in vitro* applications.

The process of blood coagulation, thrombosis, is triggered by a complex proteolytic cascade leading to the formation of thrombin. Thrombin proteolytically removes activation peptides from the A$\alpha$-chains and the B$\beta$-chains of fibrinogen, which is soluble in blood plasma, initiating insoluble fibrin formation. The formation of thrombin from prothrombin is catalyzed by factor Xa.

Anticoagulation currently is achieved by the administration of heparins and coumarins. Parenteral pharmacological control of coagulation and thrombosis is based on inhibition of thrombin through the use of heparins. Heparins act indirectly on thrombin by accelerating the inhibitory effect of endogenous antithrombin III (the main physiological inhibitor of thrombin). Because antithrombin III levels vary in plasma and because clot-bound thrombin seems resistant to this indirect mechanism, heparins can be an ineffective treatment. Because coagulation assays are believed to be associated with efficacy and with safety, heparin levels must be monitored with coagulation assays (particularly the activated partial thromboplastin time (APTT) assay). Coumarins impede the generation of thrombin by blocking the posttranslational gamma-carboxylation in the synthesis of prothrombin and other proteins of this type. Because of their mechanism of action, the effect of coumarins can only develop slowly, 6-24 hours after administration. Further, they are not selective anticoagulants. Coumarins also require monitoring with coagulation assays (particularly the prothrombin time (PT) assay).

Recently, interest has grown in small synthetic molecules which demonstrate potent direct inhibition of thrombin and factor Xa. See, Joseph P. Vacca (Annette M. Doherty Section Editor), Annual Reports in Medicinal Chemistry, (1998), 33, 81-90.

US-A-5,576,343 (Nagahara et. al.) discloses aromatic amidine derivatives which demonstrate factor Xa inhibition activity.

Although the heparins and coumarins are effective anticoagulants, there still exists a need for anticoagulants which act selectively on factor Xa or thrombin, and which, independent of antithrombin III, exert inhibitory action shortly after administration, preferably by an oral route, and do not interfere with lysis of blood clots, as required to maintain hemostasis.

The present invention is directed to the discovery that the amides of the present invention, as defined below, are potent inhibitors of factor Xa which may have high bioavailability following oral administration.

According to the invention there is provided a compound of formula I

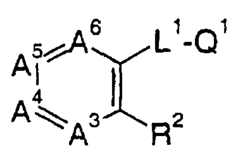

I

(or a pharmaceutically acceptable salt thereof) wherein:

$A^3$, $A^4$, $A^5$ and $A^6$, together with the two carbons to which they are attached, complete a substituted heteroaromatic ring in which

(a) one of $A^3$, $A^4$, $A^5$ and $A^6$ is N, and each of the others is $CR^3$, $CR^4$, $CR^5$ or $CR^6$, respectively; or
(b) two non-adjacent residues of $A^3$, $A^4$, $A^5$ and $A^6$ are each N, and each of the others is $CR^3$, $CR^4$, $CR^5$ or $CR^6$, respectively; wherein

each of $R^3$, $R^4$, $R^5$ and $R^6$ is independently hydrogen or methyl, or one of $R^3$, $R^4$, $R^5$ and $R^6$ attached to a carbon

which is not bonded to an N-atom is chloro and the others are hydrogen;
$L^1$ is -CO-NH- such that $-L^1-Q^1$ is $-CO-NH-Q^1$;
$Q^1$ is 2-pyridinyl (which bears a methyl, methoxy, methylthio, fluoro or chloro substituent at the 5-position), 3-pyridinyl (which bears a methyl, fluoro or chloro substituent at the 6-position), 2-pyrimidinyl (which may bear a methyl, fluoro or chloro substituent at the 5-position), 3-pyridazinyl (which may bear a methyl, fluoro or chloro substituent at the 6-position) or 2-benzothiazolyl (which may bear a methyl, fluoro, chloro or bromo substituent at the 6-position);
$R^2$ is $-L^2-Q^2$ in which $-L^2-$ is -NH-CO-, -NH-CO-X-, -NH-CO-O-X-, -NH-CO-NH-X- or $-NH-CH_2-$; and $Q^2$ is $Q^{2A}$, $Q^{2B}$, $Q^{2C}$, $Q^{2D}$, $Q^{2E}$ or $Q^{2F}$ wherein X is a single bond or methylene and the values of $L^2$ and $Q^2$ are together selected from $-NH-CO-X-Q^{2A}$, $-NH-CO-O-X-Q^{2A}$, $-NH-CO-NH-X-Q^{2A}$, $-NH-CH_2-Q^{2A}$, $-NH-CO-X-Q^{2B}$, $-NH-CO-Q^{2C}$, $-NH-CO-Q^{2D}$, $-NH-CO-Q^{2E}$ and $-NH-CO-Q^{2F}$ in which:

$Q^{2A}$ (showing the $L^2$ to which it is attached) is

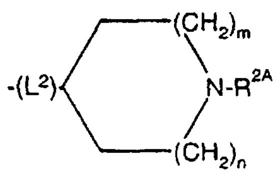

in which
each of m and n independently is 0 or 1, and
$R^{2A}$ is hydrogen, t-butyl, methylsulfonyl, $-CHR^yR^z$, $-CHR^wR^x$, or 4-pyridinyl (which is unsubstituted or bears a substituent $R^v$ at the 2- or 3-position) wherein

$R^v$ is methyl, hydroxymethyl, {(1-2C)alkoxy}carbonyl; cyano, carbamoyl, thiocarbamoyl, or N-hydroxyamidino;

each of $R^w$ and $R^x$ independently is hydrogen or (1-3C)normal alkyl; or $-CHR^wR^x$ is 2-indanyl or (showing the nitrogen to which it is attached) is

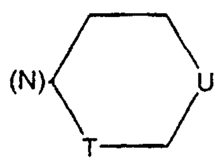

in which T is a single bond or methylene and U is methylene, ethylene, oxy, $-S(O)_q-$ (wherein q is 0, 1 or 2) or imino (which may bear a methyl substituent), or T is ethan-1,1-diyl and U is a single bond or methylene;
$R^y$ is hydrogen or methyl; and
$R^z$ is isopropyl, t-butyl, (3-6C)cycloalkyl, phenyl (which is unsubstituted or bears one or more substituents independently selected from halo, methyl, methoxy and hydroxy), 4-quinolinyl or heteroaryl (which heteroaryl is a 5-membered aromatic ring which includes one to four heteroatoms selected from sulfur, oxygen and nitrogen or is a 6-membered aromatic ring which includes one to three nitrogen atoms, wherein the heteroaryl is attached at carbon and may bear one or more methyl substituents on carbon or nitrogen);
$Q^{2B}$ is 1-piperazinyl which bears at the 4-position the group $R^{2A}$ (defined as above);
$Q^{2C}$ is 3,4-didehydropiperidin-4-yl which bears at the 1-position the group $R^{2A}$ (defined as above);
$Q^{2D}$ is cyclohexyl which bears at the 4-position the group $-NR^sR^t$ in which each of $R^s$ and $R^t$ independently is hydrogen or methyl or $R^s$ and $R^t$ together are trimethylene or tetramethylene;
$Q^{2E}$ is 1-piperidinyl which bears at the 4-position the group $-NR^sR^t$ (defined as above); and
$Q^{2F}$ (showing the $L^2$ to which it is attached) is

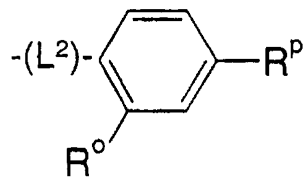

in which $R^o$ is hydrogen, halo, (1-6C)alkyl, hydroxy, (1-4C)alkoxy, benzyloxy or (1-4C)alkylthio; and $R^p$ is 1-hydroxyethyl, 1-hydroxy-1-methylethyl, 1-methoxy-1-methylethyl, 4-piperidinyl, 4-pyridinyl, dimethylaminosulfonyl or -J-$R^q$ in which J is a single bond, methylene, carbonyl, oxy, -$S(O)_q$- (wherein q is 0, 1 or 2), or -$NR^r$- (wherein $R^r$ is hydrogen or methyl); and $R^q$ is (1-6C)alkyl, phenyl, 3-pyridyl or 4-pyridyl.

As used herein, the expression a compound of formula I or the expression a compound of the invention includes the compound and any conventional prodrug thereof, as well as a pharmaceutically acceptable salt of said compound or prodrug.

A pharmaceutically acceptable salt of an antithrombotic agent of the instant invention includes one which is an acid-addition salt made from a basic compound of formula I and an acid which provides a pharmaceutically acceptable anion, as well as a salt which is made from an acidic compound of formula I and a base which provides a pharmaceutically acceptable cation. Thus, a salt of a novel compound of formula I as provided herein made with an acid or base which affords a pharmaceutically acceptable counterion provides a particular aspect of the invention. Examples of such acids and bases are provided hereinbelow.

As an additional aspect of the invention there is provided a pharmaceutical formulation comprising in association with a pharmaceutically acceptable carrier, diluent or excipient, a novel compound of formula I (or a pharmaceutically acceptable salt thereof) as provided in any of the descriptions herein.

In addition, there is provided the use of a factor Xa inhibiting compound of formula I (or prodrug or salt) as described herein as an active ingredient in the manufacture of a medicament for use in producing an anticoagulant or antithrombotic effect.

The present invention further provides for the use of a compound of formula I having any of the definitions herein for the manufacture of a medicament for inhibiting factor Xa in a mammal.

In addition, there is provided the use of a factor Xa inhibiting compound of formula I having any of the definitions herein for the manufacture of a medicament for treatment of a thromboembolic disorder.

As an additional feature of the invention there is provided a pharmaceutical formulation comprising in association with a pharmaceutically acceptable carrier, diluent or excipient, a prodrug of a factor Xa inhibiting compound of formula I (or of a pharmaceutically acceptable salt thereof) as provided in any of the descriptions herein.

In this specification, the following definitions are used, unless otherwise described: Halo is fluoro, chloro, bromo or iodo. Alkyl, alkoxy, etc. denote both straight and branched groups; but reference to an individual radical such as "propyl" embraces only the straight chain ("normal") radical, a branched chain isomer such as "isopropyl" being specifically denoted.

Particular values are listed below for radicals, substituents, and ranges, for illustration only, and they do not exclude other defined values or other values within defined ranges for the radicals and substituents.

For an alkyl group or the alkyl portion of an alkyl containing group such as, for example alkoxy, a particular value for (1-2C)alkyl is methyl or ethyl, and more particularly is methyl; for (1-3C)normal alkyl is methyl, ethyl or propyl; for (1-4C)alkyl is methyl, ethyl, propyl, isopropyl, butyl, isobutyl, or t-butyl, and more particularly is methyl, isopropyl, butyl or t-butyl; for (1-6C)alkyl is methyl, ethyl, propyl, butyl, pentyl or hexyl, and more particularly is methyl, butyl, or hexyl. A particular value for (3-6C)cycloalkyl is cyclopropyl, cyclobutyl, cyclopenytyl or cyclohexyl. A particular value for halo is bromo or chloro, and more particularly is chloro.

A particular value for $Q^1$ is 5-chloropyridin-2-yl or 6-chloropyridazin-3-yl. A particular value for $R^2$ is (1-isopropylpiperidin-4-ylcarbonyl)amino, (1-cyclohexyl-piperidin-4-ylcarbonyl)amino, [1-(tetrahydropyran-4-yl)piperidin-4-ylcarbonyl]amino, or [1-(4-pyridinyl)piperidin-4-ylmethyl]amino. A particular set of values for $A^3$-$A^6$ is that $A^3$ is N and each of $A^4$-$A^6$ is $CR^4$-$CR^6$ in which each of $R^4$-$R^6$ is hydrogen or $R^4$ and $R^6$ are each hydrogen and $R^5$ is chloro. Another set of values for $A^3$-$A^6$ is that $A^6$ is N and each of $A^3$-$A^5$ is $CR^3$-$CR^5$ in which each of $R^3$-$R^5$ is hydrogen or $R^3$ and $R^4$ are each hydrogen and $R^5$ is methyl.

A particular species is one those listed below as example 6, 8, 14, 15 or 17.

It will be appreciated that certain compounds of formula I (or salts or prodrugs, etc.) may exist in, and be isolated in, isomeric forms, including tautomeric forms, cis- or trans-isomers, as well as optically active, racemic, or diastereomeric forms. It is to be understood that the present invention encompasses a compound of formula I in any

of the tautomeric forms or as an a mixture thereof; or as a mixture of diastereomers, as well as in the form of an individual diastereomer, and that the present invention encompasses a compound of formula I as a mixture of enantiomers, as well as in the form of an individual enantiomer, any of which mixtures or form possesses inhibitory properties against factor Xa, it being well known in the art how to prepare or isolate particular forms and how to determine inhibitory properties against factor Xa by standard tests including those described below.

In addition, a compound of formula I (or salt or prodrug, etc.) may exhibit polymorphism or may form a solvate with water or an organic solvent. The present invention also encompasses any such polymorphic form, any solvate or any mixture thereof.

A prodrug of a compound of formula I may be one formed in a conventional manner with a functional group of the compound, such as with an amino, hydroxy or carboxy group.

A compound of formula I may be prepared by processes which include processes known in the chemical art for the production of structurally analogous compounds or by a novel process described herein. A process for the preparation of a compound of formula I (or a pharmaceutically acceptable salt thereof) and novel intermediates for the manufacture of a compound of formula I as defined above provide further features of the invention and are illustrated by the following procedures in which the meanings of the generic radicals are as defined above, unless otherwise specified. It will be recognized that it may be preferred or necessary to prepare a compound of formula I in which a functional group is protected using a conventional protecting group, then to remove the protecting group to provide the compound of formula I.

Thus, there is provided a process for preparing a compound of formula I (or a pharmaceutically acceptable salt thereof) as provided in any of the above descriptions which is selected from any of those described in the examples, including the following.

(A) For a compound of formula I in which $-L^2-Q^2$, is $-NH-CO-Q^2$, $-NH-CO-X-Q^2$, $-NH-CO-O-X-Q^2$ or $-NH-CO-NH-X-Q^2$, acylating an amine of formula II,

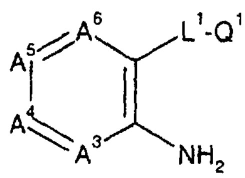

II

using a corresponding acid of formula $HO-CO-Q^2$, $HO-CO-X-Q^2$, $HO-CO-O-X-Q^2$, or $HO-CO-NH-X-Q^2$, or an activated derivative thereof. Typical activated derivatives include the acid halides, activated esters, including 4-nitrophenyl esters and those derived from coupling reagents, as well as (when the product is a urea) isocyanates. Typical procedures include those described at example 2-B, example 3-B and example 9-A.

(B) For a compound of formula I in which $-L^2-Q^2$ is $-NH-CH_2-Q^2$, and (preferably) at least one of $A^3$ and $A^5$ is N, substituting the group $Y^a$ of a compound of formula III

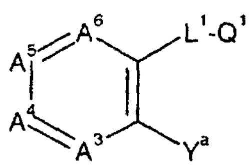

III

in which $Y^a$ is a conventional leaving group for nucleophilic aromatic substitution with an amine of formula $NH_2-CH_2-Q^2$. As used herein, a leaving group "$Y^a$" is a moiety which is displaced in an aromatic (or heteroaromatic) nucleophilic substitution reaction, for example a halo group (such as fluoro or chloro), an alkoxy group (such as methoxy), a sulfonate ester group (such as methylsulfonyloxy, p-toluylsulfonyloxy or trifluoromethylsulfonyloxy), or the reactive species derived from treating an alcohol with triphenylphospine, diethyl azodicarboxylate and triethyl amine (in a Mitsunobu reaction). The substitution may be carried out by heating a mixture of the reagents in a polar solvent, for example in ethanol in a sealed tube as described at example 14-B or in refluxing pyridine as described at example 23-B.

(C) Acylating an amine of formula $H_2N-Q^1$, or a deprotonated derivative thereof, using an acid of formula IV, or an activated derivative thereof.

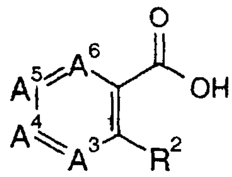

IV

Typical deprotonated derivatives of the amine $H_2N-Q^1$ include, for example, that derived from treatment of the amine with an organomagnesium reagent, for example, with allylmagnesium bromide or methylmagnesium bromide. Typical activated derivatives include the acid halides, activated esters, including 4-nitrophenyl esters and those derived from coupling reagents. A typical procedure is for example one using an acid chloride as described in example 4

For a compound of formula I in which $R^2$ is of the form $-NH-CO-Q^2$, the activated acid may be a [1,3]oxazine of formula IVa,

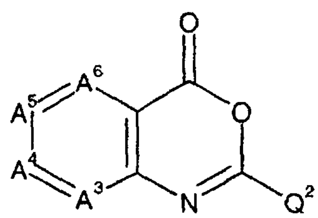

IVa

wherein $Q^2$ represents, for example, $Q^{2A}$, $Q^{2B}$, $Q^{2C}$, $Q^{2D}$, $Q^{2E}$ or $Q^{2F}$. A typical procedure is one such as described at example 21-H.

For a compound of formula I in which $R^2$ is of the form $-NH-CH_2-Q^2$, the activated acid may be an anhydride of formula IVb,

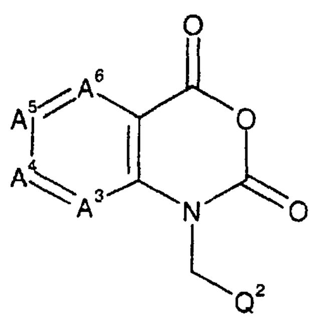

IVb

wherein $Q^2$ represents $Q^{2A}$. A typical procedure is that described at example 1-C.

(D) For a compound of formula I in which $R^2$ is $-NH-CH_2-Q^{2A}$, alkylating an amine of formula II directly, using a compound of formula $Y-CH_2-Q^{2A}$, or (preferably) indirectly by reductive alkylation using an aldehyde of formula $Q^{2A}-CHO$. In the reductive alkylation the intermediate imine of formula V or acid addition salt thereof (which provide a further aspect of the invention),

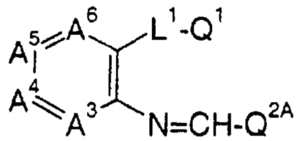

V

may be formed in situ and reduced directly, or may be isolated prior to reduction, for example as described at example 13-D where the reduction is carried out using borane trimethylamine complex in glacial acetic acid.

(E) For a compound of formula I in which $R^2$ is -NH-CO-O-X-$Q^{2A}$, or -NH-CO-NH-X-$Q^{2A}$, acylating an alcohol of formula HO-X-$Q^{2A}$ or an amine of formula $NH_2$-X-$Q^{2A}$, using an activated derivative of an acid of formula VI,

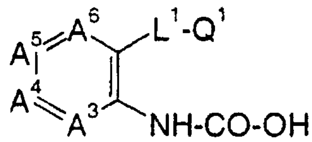

VI

particularly, the corresponding isocyanate or 4-nitrophenyl ester.

(F) For a compound of formula I in which $R^2$ is -NH-CO-X-$Q^{2B}$ in which X is a single bond, acylating at the 1-position a piperazine of formula H-$Q^{2B}$, using an activated derivative of an acid of formula VI, particularly, the corresponding isocyanate or 4-nitrophenyl ester.

(G) For a compound of formula I in which $R^2$ is -NH-CO-X-$Q^{2B}$ in which X is methylene, alkylating at the 1-position a piperazine of formula H-$Q^{2B}$, using an alkylating agent of formula VII

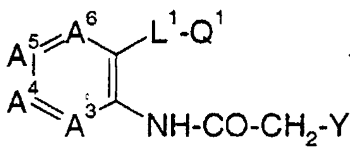

VII

in which Y is a leaving group.

(H) For a compound of formula I in which $R^{2A}$ is methylsulfonyl, substituting the amino nitrogen of a corresponding compound of formula I in which $R^{2A}$ is hydrogen using an activated derivative of methanesulfonic acid, for example using methanesulfonyl chloride in the presence of added base.

(I) For a compound of formula I in which $R^{2A}$ is -$CHR^yR^z$ or -$CHR^wR^x$, alkylating the amino nitrogen of a corresponding compound of formula I in which $R^{2A}$ is hydrogen using an alkylating agent of formula Y-$CHR^yR^z$ or Y-$CHR^wR^x$ or, preferably, reductively alkylating the amine using a compound of formula $R^y$-CO-$R^z$ or $R^w$CO-R-$^x$. The direct alkylation may be completed in a polar solvent in the presence of a base. The reductive alkylation conveniently is carried out, for example, using sodium cyanoborohydride in methanol/acetic acid as described at example 13-E or using sodium triacetoxyborohydride in an inert solvent such as 1,2-dichloroethane along with an excess of the carbonyl compound and glacial acetic acid as described at example 6-C.

(J) For a compound of formula I in which $R^{2A}$ is 4-pyridinyl (which is unsubstituted or bears a substituent $R^v$ at the 2- or 3-position), substituting the amino nitrogen of a corresponding compound of formula I in which $R^{2A}$ is hydrogen using a corresponding pyridine reagent bearing a leaving group Y at the 4-position, for example with a 4-chloropyridine in ethanol as described at example 18.

(K) For a compound of formula I in which $R^{2A}$ is 4-pyridinyl in which $R^v$ is alkoxycarbonyl, esterifying a corresponding compound of formula I in which $R^v$ is carboxy.

(L) For a compound of formula I in which $R^{2A}$ is 4-pyridinyl in which $R^v$ is hydroxymethyl, reducing the ester of a corresponding compound of formula I in which $R^v$ is alkoxycarbonyl.

(M) For a compound of formula I in which $R^{2A}$ is 4-pyridinyl in which $R^v$ is carbamoyl, amidating the ester of a corresponding compound of formula I in which $R^v$ is alkoxycarbonyl.

(N) For a compound of formula I in which $R^{2A}$ is 4-pyridinyl in which $R^v$ is thiocarbamoyl, adding $H_2S$ to the

nitrile of a corresponding compound of formula I in which $R^v$ is cyano.

(O) For a compound of formula I in which $R^{2A}$ is 4-pyridinyl in which $R^v$ is N-hydroxyamidino, adding $H_2NOH$ to the nitrile of a corresponding compound of formula I in which $R^v$ is cyano. The addition may be direct or indirect, such as via an imidate ester or by treating a compound in which $R^v$ is thiocarbamoyl with methyl iodide to form a thioimidate ester, then treatment with hydroxylamine.

(P) For a compound of formula I in which $R^{2A}$ is 4-pyridinyl in which $R^v$ is carboxy, decomposing the ester of a corresponding compound of formula I in which $R^v$ is alkoxycarbonyl.

(Q) For a compound of formula I in which $-NR^sR^t$ is other than amino, alkylating a corresponding compound of formula I in which $-NR^sR^t$ is amino using a conventional method. When $R^s$ and $R^t$ together are trimethylene or tetramethylene, a difunctional alkylating agent, such as 1,3-dibromopropane or 1,4-dibromobutane is preferred.

(R) For a compound of formula I which bears $-NR^sR^t$, reductively alkylating $H-NR^sR^t$ using a corresponding compound but in which the carbon to bear the $-NR^sR^t$ group bears an oxo group, for example, using a procedure similar to one of procedure (I) above.

(S) For a compound of formula I in which $R^p$ is 1-hydroxy-1-methylethyl, adding a methyl group to the carbonyl group of a corresponding compound of formula I in which $R^p$ is acetyl using an organometallic reagent such as, for example, methylmagnesium bromide.

(T) For a compound of formula I in which $R^p$ is 1-methoxy-1-methylethyl, treating a corresponding compound of formula I in which $R^p$ is 1-hydroxy-1-methylethyl with methanol and an acid catalyst.

Whereafter, for any of the above procedures, when a functional group is protected using a protecting group, removing the protecting group.

Whereafter, for any of the above procedures, when a pharmaceutically acceptable salt of a compound of formula I is required, it is obtained by reacting the basic form of a basic compound of formula I with an acid affording a physiologically acceptable counterion or the acidic form of an acidic compound of formula I with a base affording a physiologically acceptable counterion or by any other conventional procedure.

A novel intermediate or starting material compound such as, for example, a novel compound of formula II, III, IV or VI, etc., provides a further aspect of the invention. The various starting materials may be made by processes which include processes known in the chemical art for the production of structurally analogous compounds or by a novel process described herein or one analogous thereto.

As mentioned above, a compound corresponding to a compound of formula I but in which a functional group is protected may serve as an intermediate for a compound of formula I. Accordingly, such a protected intermediate for a novel compound of formula I provides a further aspect of the invention. Thus, as one particular aspect of the invention, there is provided a compound corresponding to a novel compound of formula I as defined above in which $R^4$ is hydroxy, but in which the corresponding substituent is $-OP^p$ in place of hydroxy, wherein $P^p$ is a phenol protecting group other than (1-4C)alkyl or benzyl. Phenol protecting groups are well known in the art, for example as described in T.W. Greene and P.G.M. Wuts, "Protecting Groups in Organic Synthesis" (1991). Further, $P^p$ may denote a functionalized resin, for example as disclosed in H.V. Meyers, et al., Molecular Diversity, (1995), 1, 13-20.

As mentioned above, the invention includes a pharmaceutically acceptable salt of the factor Xa inhibiting compound defined by the above formula I. A basic compound of this invention possesses one or more functional groups sufficiently basic to react with any of a number of inorganic and organic acids affording a physiologically acceptable counterion to form a pharmaceutically acceptable salt. Acids commonly employed to form pharmaceutically acceptable acid addition salts are inorganic acids such as hydrochloric acid, hydrobromic acid, hydroiodic acid, sulfuric acid, phosphoric acid, and the like, and organic acids such as p-toluenesulfonic acid, methanesulfonic acid, oxalic acid, p-bromobenzenesulfonic acid, carbonic acid, succinic acid, citric acid, benzoic acid, acetic acid, and the like. Examples of such pharmaceutically acceptable salts thus are the sulfate, pyrosulfate, bisulfate, sulfite, bisulfite, phosphate, monohydrogenphosphate, dihydrogenphosphate, metaphosphate, pyrophosphate, chloride, bromide, iodide, acetate, propionate, decanoate, caprylate, acrylate, formate, isobutyrate, caproate, heptanoate, propiolate, oxalate, malonate, succinate, suberate, sebacate, fumarate, maleate, butyne-1,4-dioate, hexyne-1,6-dioate, benzoate, chlorobenzoate, methylbenzoate, dinitrobenzoate, hydroxybenzoate, methoxybenzoate, phthalate, sulfonate, xylenesulfonate, phenylacetate, phenylpropionate, phenylbutyrate, citrate, lactate, gamma-hydroxybutyrate, glycollate, tartrate, methanesulfonate, propanesulfonate, naphthalene-1-sulfonate, naphthalene-2-sulfonate, mandelate, and the like. Preferred pharmaceutically acceptable acid addition salts include those formed with mineral acids such as hydrochloric acid, hydrobromic acid and sulfuric acid.

For a compound of formula I which bears an acidic moiety, such as a carboxy group, a pharmaceutically acceptable salt may be made with a base which affords a pharmaceutically acceptable cation, which includes alkali metal salts (especially sodium and potassium), alkaline earth metal salts (especially calcium and magnesium), aluminum salts and ammonium salts, as well as salts made from physiologically acceptable organic bases such as triethylamine, morpholine, piperidine and triethanolamine.

If not commercially available, a necessary starting material for the preparation of a compound of formula I

may be prepared by a procedure which is selected from standard techniques of organic chemistry, including aromatic and heteroaromatic substitution and transformation, from techniques which are analogous to the syntheses of known, structurally similar compounds, and techniques which are analogous to the above described procedures or procedures described in the Examples. It will be clear to one skilled in the art that a variety of sequences is available for the preparation of the starting materials.

Selective methods of substitution, protection and deprotection are well known in the art for preparation of a compound such as one of formula II, III, IV or VI discussed above.

Generally, a basic compound of the invention is isolated best in the form of an acid addition salt. A salt of a compound of formula I formed with an acid such as one of those mentioned above is useful as a pharmaceutically acceptable salt for administration of the antithrombotic agent and for preparation of a formulation of the agent. Other acid addition salts may be prepared and used in the isolation and purification of the compounds.

As noted above, the optically active isomers and diastereomers of the compounds of formula I are also considered part of this invention. Such optically active isomers may be prepared from their respective optically active precursors by the procedures described above, or by resolving the racemic mixtures. This resolution can be carried out by derivatization with a chiral reagent followed by chromatography or by repeated crystallization. Removal of the chiral auxiliary by standard methods affords substantially optically pure isomers of the compounds of the present invention or their precursors. Further details regarding resolutions can be obtained in Jacques, et al., Enantiomers, Racemates, and Resolutions, John Wiley & Sons, 1981.

The compounds of the invention are believed to selectively inhibit factor Xa over other proteinases and nonenzyme proteins involved in blood coagulation without appreciable interference with the body's natural clot lysing ability (the compounds have a low inhibitory effect on fibrinolysis). Further, such selectivity is believed to permit use with thrombolytic agents without substantial interference with thrombolysis and fibrinolysis.

The invention in one of its aspects provides for the use of a compound of formula I for the manufacture of a medicament for inhibiting factor Xa in mammals.

In another of its aspects, the invention provides for the use of a compound of formula I for the manufacture of a medicament for treating a thromboembolic disorder.

The invention in another of its aspects provides for the use of a compound of formula I for the manufacture of a medicament for inhibiting coagulation in a mammal.

The factor Xa inhibition, coagulation inhibition and thromboembolic disorder medicament contemplated by the present method includes both medical therapeutic and/or prophylactic treatment as appropriate.

In a further embodiment, the invention relates to the manufacture of a medicament for the treatment, in a human or animal, of a condition where inhibition of factor Xa is required. The compounds of the invention are expected to be useful in mammals, including man, in treatment or prophylaxis of thrombosis and hypercoagulability in blood and tissues. Disorders in which the compounds have a potential utility are in treatment or prophylaxis of thrombosis and hypercoagulability in blood and tissues. Disorders in which the compounds have a potential utility, in treatment and/or prophylaxis, include venous thrombosis and pulmonary embolism, arterial thrombosis, such as in myocardial ischemia, myocardial infarction, unstable angina, thrombosis-based stroke and peripheral arterial thrombosis. Further, the compounds have expected utility in the treatment or prophylaxis of atherosclerotic disorders (diseases) such as coronary arterial disease, cerebral arterial disease and peripheral arterial disease. Further, the compounds are expected to be useful together with thrombolytics in myocardial infarction. Further, the compounds have expected utility in prophylaxis for reocclusion after thrombolysis, percutaneous transluminal angioplasty (PTCA) and coronary bypass operations. Further, the compounds have expected utility in prevention of rethrombosis after microsurgery. Further, the compounds are expected to be useful in anticoagulant treatment in connection with artificial organs, including joint replacement, and cardiac valves. Further, the compounds have expected utility in anticoagulant treatment in hemodialysis and disseminated intravascular coagulation. A further expected utility is in rinsing of catheters and mechanical devices used in patients *in vivo,* and as an anticoagulant for preservation of blood, plasma and other blood,products *in vitro.* Still further, the compounds have expected utility in other diseases where blood coagulation could be a fundamental contributing process or a source of secondary pathology, such as cancer, including metastasis, inflammatory diseases, including arthritis, and diabetes. The anti-coagulant compound is administered orally or parenterally, e.g. by intravenous infusion (iv), intramuscular injection (im) or subcutaneously (sc).

The specific dose of a compound administered according to this invention to obtain therapeutic and/or prophylactic effects will, of course, be determined by the particular circumstances surrounding the case, including, for example, the compound administered, the rate of administration, the route of administration, and the condition being treated.

A typical daily dose for each of the above utilities is between about 0.01 mg/kg and about 1000 mg/kg. The dose regimen may vary e.g. for prophylactic use a single daily dose may be administered or multiple doses such as 3 or 5 times daily may be appropriate. In critical care situations a compound of the invention is administered by iv infusion at a rate between about 0.01 mg/kg/h and about 20 mg/kg/h and preferably between about 0.1 mg/kg/h and about 5

mg/kg/h.

The use of this invention also is practiced in conjunction with a clot lysing agent e.g. tissue plasminogen activator (t-PA), modified t-PA, streptokinase or urokinase. In cases when clot formation has occurred and an artery or vein is blocked, either partially or totally, a clot lysing agent is usually employed. A compound of the invention can be administered prior to or along with the lysing agent or subsequent to its use, and preferably further is administered along with aspirin to prevent the reoccurrence of clot formation.

The use of this invention is also practiced in conjunction with a platelet glycoprotein receptor (IIb/IIIa) antagonist, that inhibits platelet aggregation. A compound of the invention can be administered prior to or along with the IIb/IIIa antagonist or subsequent to its use to prevent the occurrence or reoccurrence of clot formation.

The use of this invention is also practiced in conjunction with aspirin. A compound of the invention can be administered prior to or along with aspirin or subsequent to its use to prevent the occurrence or reoccurrence of clot formation. As stated above, preferably a compound of the present invention is administered in conjunction with a clot lysing agent and aspirin.

This invention also provides a pharmaceutical composition for use in the above described therapeutic method. A pharmaceutical composition of the invention comprises an effective factor Xa inhibiting amount of a compound of formula I in association with a pharmaceutically acceptable carrier, excipient or diluent.

The active ingredient in such formulations comprises from 0.1 percent to 99.9 percent by weight of the formulation. By "pharmaceutically acceptable" it is meant the carrier, diluent or excipient must be compatible with the other ingredients of the formulation and not deleterious to the recipient thereof.

For oral administration the antithrombotic compound is formulated in gelatin capsules or tablets which may contain excipients such as binders, lubricants, disintegration agents and the like. For parenteral administration the antithrombotic is formulated in a pharmaceutically acceptable diluent e.g. physiological saline (0.9 percent), 5 percent dextrose, Ringer's solution and the like.

The compound of the present invention can be formulated in unit dosage formulations comprising a dose between about 0.1 mg and about 1000 mg. Preferably the compound is in the form of a pharmaceutically acceptable salt such as for example the sulfate salt, acetate salt or a phosphate salt. An example of a unit dosage formulation comprises 5 mg of a compound of the present invention as a pharmaceutically acceptable salt in a 10 mL sterile glass ampoule. Another example of a unit dosage formulation comprises about 10 mg of a compound of the present invention as a pharmaceutically acceptable salt in 20 mL of isotonic saline contained in a sterile ampoule.

The compounds can be administered by a variety of routes including oral, rectal, transdermal, subcutaneous, intravenous, intramuscular, and intranasal. The compounds of the present invention are preferably formulated prior to administration.

The present pharmaceutical compositions are prepared by known procedures using well known and readily available ingredients. The compositions of this invention may be formulated so as to provide quick, sustained, or delayed release of the active ingredient after administration to the patient by employing procedures well known in the art. In making the compositions of the present invention, the active ingredient will usually be admixed with a carrier, or diluted by a carrier, or enclosed within a carrier which may be in the form of a capsule, sachet, paper or other container. When the carrier serves as a diluent, it may be a solid, semi-solid or liquid material which acts as a vehicle, excipient or medium for the active ingredient. Thus, the compositions can be in the form of tablets, pills, powders, lozenges, sachets, cachets, elixirs, suspensions, emulsions, solutions, syrups, aerosols, (as a solid or in a liquid medium), soft and hard gelatin capsules, suppositories, sterile injectable solutions, sterile packaged powders, and the like.

The following formulation examples are illustrative only and are not intended to limit the scope of the invention in any way. "Active ingredient," of course, means a compound according to formula I or a pharmaceutically acceptable salt or solvate thereof.

| Formulation 1: Hard gelatin capsules are prepared using the following ingredients: | |
|---|---|
| | Quantity (mg/capsule) |
| Active ingredient | 250 |
| Starch, dried | 200 |
| Magnesium stearate | 10 |
| Total | 460 mg |

| Formulation 2: A tablet is prepared using the ingredients below: | |
|---|---|
| | Quantity (mg/tablet) |
| Active ingredient | 250 |
| Cellulose, microcrystalline | 400 |
| Silicon dioxide, fumed | 10 |
| Stearic acid | 5 |
| Total | 665 mg |

The components are blended and compressed to form tablets each weighing 665 mg.

| Formulation 3: An aerosol solution is prepared containing the following components: | |
|---|---|
| | Weight |
| Active ingredient | 0.25 |
| Ethanol | 29.75 |
| Propellant 22 (Chlorodifluoromethane) | 70.00 |
| Total | 100.00 |

The active compound is mixed with ethanol and the mixture added to a portion of the propellant 22, cooled to -30 °C and transferred to a filling device. The required amount is then fed to a stainless steel container and diluted with the remainder of the propellant. The valve units are then fitted to the container.

| Formulation 4: Tablets, each containing 60 mg of active ingredient, are made as follows: | |
|---|---|
| Active ingredient | 60 mg |
| Starch | 45 mg |
| Microcrystalline cellulose | 35 mg |
| Polyvinylpyrrolidone (as 10% solution in water) | 4 mg |
| Sodium carboxymethyl starch | 4.5 mg |
| Magnesium stearate | 0.5 mg |
| Talc | 1 mg |
| Total | 150 mg |

The active ingredient, starch and cellulose are passed through a No. 45 mesh U.S. sieve and mixed thoroughly. The aqueous solution containing polyvinylpyrrolidone is mixed with the resultant powder, and the mixture then is passed through a No. 14 mesh U.S. sieve. The granules so produced are dried at 50 °C and passed through a No. 18 mesh U.S. Sieve. The sodium carboxymethyl starch, magnesium stearate and talc, previously passed through a No. 60 mesh U.S. sieve, are then added to the granules which, after mixing, are compressed on a tablet machine to yield tablets each weighing 150 mg.

| Formulation 5: Capsules, each containing 80 mg of active ingredient, are made as follows: | |
|---|---|
| Active ingredient | 80 mg |
| Starch | 59 mg |
| Microcrystalline cellulose | 59 mg |
| Magnesium stearate | 2 mg |
| Total | 200 mg |

The active ingredient, cellulose, starch, and magnesium stearate are blended, passed through a No. 45 mesh U.S. sieve, and filled into hard gelatin capsules in 200 mg quantities.

| Formulation 6: Suppositories, each containing 225 mg of active ingredient, are made as follows: | |
|---|---|
| Active ingredient | 225 mg |

(continued)

| Formulation 6: Suppositories, each containing 225 mg of active ingredient, are made as follows: | |
|---|---|
| Saturated fatty acid glycerides | 2,000 mg |
| Total | 2,225 mg |

The active ingredient is passed through a No. 60 mesh U.S. sieve and suspended in the saturated fatty acid glycerides previously melted using the minimum heat necessary. The mixture is then poured into a suppository mold of nominal 2 g capacity and allowed to cool.

| Formulation-7: Suspensions, each containing 50 mg of active ingredient per 5 mL dose, are made as follows: | |
|---|---|
| Active ingredient | 50 mg |
| Sodium carboxymethyl cellulose | 50 mg |
| Syrup | 1.25 mL |
| Benzoic acid solution | 0.10 mL |
| Flavor | q.v. |
| Color | q.v. |
| Purified water to total | 5 mL |

The active ingredient is passed through a No. 45 mesh U.S. sieve and mixed with the sodium carboxymethyl cellulose and syrup to form a smooth paste. The benzoic acid solution, flavor and color are diluted with a portion of the water and added, with stirring. Sufficient water is then added to produce the required volume.

| Formulation 8: An intravenous formulation may be prepared as follows: | |
|---|---|
| Active ingredient | 100 mg |
| Isotonic saline | 1,000 mL |

The solution of the above ingredients generally is administered intravenously to a subject at a rate of 1 mL per minute.

The ability of a compound of the present invention to be an effective and orally active factor Xa inhibitor may be evaluated in one or more of the following assays or in other standard assays known to those in the art.

The inhibition by a compound of the inhibition of a serine protease of the human blood coagulation system or of the fibrinolytic system, as well as of trypsin, is determined in vitro for the particular enzyme by measuring its inhibitor binding affinity in an assay in which the enzyme hydrolyzes a particular chromogenic substrate, for example as described in Smith, G.F.; Gifford-Moore, D.; Craft, T.J.; Chirgadze, N.; Ruterbories, K.J.; Lindstrom, T.D.; Satterwhite, J.H. Efegatran: A New Cardiovascular Anticoagulant. *New Anticoagulants for the Cardiovascular Patient*; Pifarre, R., Ed.; Hanley & Belfus, Inc.: Philadelphia, 1997; pp. 265-300. The inhibitor binding affinity is measured as apparent association constant Kass which is the hypothetical equilibrium constant for the reaction between enzyme and the test inhibitor compound (I).

$$\text{Enzyme} + \text{I} \rightleftharpoons \text{Enzyme-I}$$

$$\text{Kass} = \frac{[\text{Enzyme-I}]}{[(\text{Enzyme}) \times (\text{I})]}$$

Conveniently, enzyme inhibition kinetics are performed in 96-well polystyrene plates and reaction rates are determined from the rate of hydrolysis of appropriate p-nitroanilide substrates at 405 nm using a Thermomax plate reader from Molecular Devices (San Francisco, CA). The same protocol is followed for all enzymes studied: 50 µL buffer (0.03 M Tris, 0.15 M NaCl pH 7) in each well, followed by 25 µL of inhibitor solution (in 100% methanol, or in 50% v:v aqueous methanol) and 25 µL enzyme solution; within two minutes, 150 µL aqueous solution of chromogenic substrate (0.25 mg/mL) is added to start the enzymatic reaction. The rates of chromogenic substrate hydrolysis reactions provide a linear relationship with the enzymes studied such that free enzyme can be quantitated in reaction

mixtures. Data is analyzed directly as rates by the Softmax program to produce [free enzyme] calculations for tight-binding Kass determinations. For apparent Kass determinations, 1.34 nM human factor Xa is used to hydrolyze 0.18 mM BzIle-Glu-Gly-Arg-pNA; 5.9 nM human thrombin or 1.4 nM bovine trypsin is used to hydrolyze 0.2 mM BzPhe-Val-Arg-pNA; 3.4 nM human plasmin is used with 0.5 mM HD-Val-Leu-Lys-pNA; 1.2 nM human nt-PA is used with 0.81 mM HD-Ile-Pro-Arg-pNA; and 0.37 nM urokinase is used with 0.30 mM pyro-gfsGlu-Gly-Arg-pNA.

Kass is calculated for a range of concentrations of test compounds and the mean value reported in units of liter per mole. In general, a factor Xa inhibiting compound of formula I of the instant invention, as exemplified herein, exhibits a Kass of 0.1 to 0.5 x $10^6$ L/mole or much greater.

The factor Xa inhibitor preferably should spare fibrinolysis induced by urokinase, tissue plasminogen activator (t-PA) and streptokinase. This would be important to the therapeutic use of such an agent as an adjunct to streptokinase, tp-PA or urokinase thrombolytic therapy and to the use of such an agent as an endogenous fibrinolysis-sparing (with respect to t-PA and urokinase) antithrombotic agent. In addition to the lack of interference with the amidase activity of the fibrinolytic proteases, such fibrinolytic system sparing can be studied by the use of human plasma clots and their lysis by the respective fibrinolytic plasminogen activators.

Materials

Dog plasma is obtained from conscious mixed-breed hounds (either sex Butler Farms, Clyde, New York, U. S.A.) by venipuncture into 3.8 percent citrate. Fibrinogen is prepared from fresh dog plasma and human fibrinogen is prepared from in-date ACD human blood at the fraction I-2 according to previous procedures and specification. Smith, Biochem. J., 185, 1-11 (1980; and Smith, et al., Biochemistry, 11, 2958-2967, (1972). Human fibrinogen (98 percent pure/plasmin free) is from American Diagnostica, Greenwich, Connecticut. Radiolabeling of fibrinogen I-2 preparations is performed as previously reported. Smith, et al., Biochemistry, 11, 2958-2967, (1972). Urokinase is purchased from Leo Pharmaceuticals, Denmark, as 2200 Ploug units/vial. Streptokinase is purchased from Hoechst-Roussel Pharmaceuticals, Somerville, New Jersey.

Methods - Effects on Lysis of Human Plasma Clots by t-PA

Human plasma clots are formed in micro test tubes by adding 50 μL thrombin (73 NIH unit/mL) to 100 μL human plasma which contains 0.0229 μCi 125-iodine labeled fibrinogen. Clot lysis is studied by overlaying the clots with 50 μL of urokinase or streptokinase (50, 100, or 1000 unit/mL) and incubating for 20 hours at room temperature. After incubation the tubes are centrifuged in a Beckman Microfuge. 25 μL of supernate is added into 1.0 mL volume of 0.03 M tris/0.15 M NaCl buffer for gamma counting. Counting controls 100 percent lysis are obtained by omitting thrombin (and substituting buffer). The factor Xa inhibitors are evaluated for possible interference with fibrinolysis by including the compounds in the overlay solutions at 1, 5, and 10 μg/mL concentrations. Rough approximations of $IC_{50}$ values are estimated by linear extrapolations from data points to a value which would represent 50 percent of lysis for that particular concentration of fibrinolytic agent.

Anticoagulant Activity

Materials

Dog plasma and rat plasma are obtained from conscious mixed-breed hounds (either sex, Butler Farms, Clyde, New York, U.S.A.) or from anesthetized male Sprague-Dawley rats (Harlan Sprague-Dawley, Inc., Indianapolis, Indiana, U.S.A.) by venipuncture into 3.8 percent citrate. Fibrinogen is prepared from in-date ACD human blood as the fraction I-2 according to previous procedures and specifications. Smith, Biochem. J., 185, 1-11 (1980); and Smith, et al., Biochemistry, 11, 2958-2967 (1972). Human fibrinogen is also purchased as 98 percent pure/plasmin free from American Diagnostica, Greenwich, Connecticut. Coagulation reagents Actin, Thromboplastin, Innovin and Human plasma are from Baxter Healthcare Corp., Dade Division, Miami, Florida. Bovine thrombin from Parke-Davis (Detroit, Michigan) is used for coagulation assays in plasma.

Methods

Anticoagulation Determinations

Coagulation assay procedures are as previously described. Smith, et al., Thrombosis Research, 50, 163-174 (1988). A CoAScreener coagulation instrument (American LABor, Inc.) is used for all coagulation assay measurements. The prothrombin time (PT) is measured by adding 0.05 mL saline and 0.05 mL Thromboplastin-C reagent or recombinant human tissue factor reagent (Innovin) to 0.05 mL test plasma. The activated partial thromboplastin time (APTT) is measured by incubation of 0.05 mL test plasma with 0.05 mL Actin reagent for 120 seconds followed by 0.05 mL $CaCl_2$ (0.02 M). The thrombin time (TT) is measured by adding 0.05 mL saline and 0.05 mL thrombin (10 NIH units/mL) to 0.05 mL test plasma. The compounds of formula I are added to human or animal plasma over a wide range of concentrations to determine prolongation effects on the APTT, PT, and TT assays. Linear extrapolations are performed to estimate the concentrations required to double the clotting time for each assay.

Animals

Male Sprague Dawley rats (350-425 gm, Harlan Sprague Dawley Inc., Indianapolis, IN) are anesthetized with xylazine (20 mg/kg, s.c.) and ketamine (120 mg/kg, s.c.) and maintained on a heated water blanket (37 °C). The jugular vein(s) is cannulated to allow for infusions.

Arterio-Venous shunt model

The left jugular vein and right carotid artery are cannulated with 20 cm lengths of polyethylene PE 60 tubing. A 6 cm center section of larger tubing (PE 190) with a cotton thread (5 cm) in the lumen, is friction fitted between the longer sections to complete the arterio-venous shunt circuit. Blood is circulated through the shunt for 15 min before the thread is carefully removed and weighed. The weight of a wet thread is subtracted from the total weight of the thread and thrombus (see J.R. Smith, Br J Pharmacol, 77:29, 1982).

$FeCl_3$ model of arterial injury

The carotid arteries are isolated via a midline ventral cervical incision. A thermocouple is placed under each artery and vessel temperature is recorded continuously on a strip chart recorder. A cuff of tubing (0.058 ID x 0.077 OD x 4 mm, Baxter Med. Grade Silicone), cut longitudinally, is placed around each carotid directly above the thermocouple. $FeCl_3$ hexahydrate is dissolved in water and the concentration (20 percent) is expressed in terms of the actual weight of $FeCl_3$ only. To injure the artery and induce thrombosis, 2.85 μL is pipetted into the cuff to bathe the artery above the thermocouple probe. Arterial occlusion is indicated by a rapid drop in temperature. The time to occlusion is reported in minutes and represents the elapsed time between application of $FeCl_3$ and the rapid drop in vessel temperature (see K.D. Kurz, Thromb. Res., 60:269, 1990).

Coagulation parameters

Plasma thrombin time (TT) and activated partial thromboplastin time (APTT) are measured with a fibrometer. Blood is sampled from a jugular catheter and collected in syringe containing sodium citrate (3.8 percent, 1 part to 9 parts blood). To measure TT, rat plasma (0.1 mL) is mixed with saline (0.1 mL) and bovine thrombin (0.1 mL, 30 U/mL in TRIS buffer; Parke Davis) at 37 °C. For APTT, plasma (0.1 mL) and APTT solution (0.1 mL, Organon Teknika) are incubated for 5 minutes (37 °C) and $CaCl_2$ (0.1 mL, 0.025 M) is added to start coagulation. Assays are done in duplicate and averaged.

Index of Bioavailability

Bioavailability studies may be conducted as follows. Compounds are administered as aqueous solutions to male Fisher rats, intravenously (iv) at 5 mg/kg via tail vein injection and orally (po) to fasted animals at 20 mg/kg by gavage. Serial blood samples are obtained at 5, 30, 120, and 240 minutes postdose following intravenous administration and at 1, 2, 4, and 6 hours after oral dosing. Plasma is analyzed for drug concentration using an HPLC procedure involving C8 Bond Elute (Varion) cartridges for sample preparation and a methanol/30 nM ammonium acetate buffer (pH 4) gradient optimized for each compound. % Oral bioavailability is calculated by the following equation:

$$\%\text{ Oral bioavailability} = \frac{\text{AUC po}}{\text{AUC iv}} \times \frac{\text{Dose iv}}{\text{Dose po}} \times 100$$

where AUC is area under the curve calculated from the plasma level of compound over the time course of the experiment following oral (AUC po) and intravenous (AUC iv) dosing.

Compounds

Compound solutions are prepared fresh daily in normal saline and are injected as a bolus or are infused starting 15 minutes before and continuing throughout the experimental perturbation which is 15 minutes in the arteriovenous shunt model and 60 minutes in the $FeCl_3$ model of arterial injury and in the spontaneous thrombolysis model. Bolus injection volume is 1 mL/kg for i.v., and 5 mL/kg for p.o., and infusion volume is 3 mL/hr.

Statistics

Results are expressed as means +/- SEM. One-way analysis of variance is used to detect statistically significant differences and then Dunnett's test is applied to determine which means are different. Significance level for rejection of the null hypothesis of equal means is $P<0.05$.

Animals

Male dogs (Beagles; 18 months - 2 years; 12-13 kg, Marshall Farms, North Rose, New York 14516) are fasted overnight and fed Purina certified Prescription Diet (Purina Mills, St. Louis, Missouri) 240 minutes after dosing. Water is available *ad libitum*. The room temperature is maintained between 66-74 °F; 45-50 percent relative humidity; and lighted from 0600-1800 hours.

Pharmacokinetic model.

Test compound is formulated immediately prior to dosing by dissolving in sterile 0.9 percent saline to a 5 mg/mL preparation. Dogs are given a single 2 mg/kg dose of test compound by oral gavage. Blood samples (4.5 mL) are taken from the cephalic vein at 0.25, 0.5, 0.75, 1, 2, 3, 4 and 6 hours after dosing. Samples are collected in citrated Vacutainer tubes and kept on ice prior to reduction to plasma by centrifugation. Plasma samples are analyzed by HPLC MS. Plasma concentration of test compound is recorded and used to calculate the pharmacokinetic parameters: elimination rate constant, Ke; total clearance, Clt; volume of distribution, $V_D$; time of maximum plasma test compound concentration, Tmax; maximum concentration of test compound of Tmax, Cmax; plasma half-life, t0.5; and area under the curve, A.U.C.; fraction of test compound absorbed, F.

Canine Model of Coronary Artery Thrombosis

Surgical preparation and instrumentation of the dogs are as described in Jackson, et al., Circulation, 82, 930-940 (1990). Mixed-breed hounds (aged 6-7 months, either sex, Butler Farms, Clyde, New York, U.S.A.) are anesthetized with sodium pentobarbital (30 mg/kg intravenously, i.v.), intubated, and ventilated with room air. Tidal volume and respiratory rates are adjusted to maintain blood $PO_2$, $PCO_2$, and pH within normal limits. Subdermal needle electrodes are inserted for the recording of a lead II ECG.

The left jugular vein and common carotid artery are isolated through a left mediolateral neck incision. Arterial blood pressure (ABP) is measured continuously with a precalibrated Millar transducer (model (MPC-500, Millar Instruments, Houston, TX, U.S.A.) inserted into the carotid artery. The jugular vein is cannulated for blood sampling during the experiment. In addition, the femoral veins of both hindlegs are cannulated for administration of test compound.

A left thoracotomy is performed at the fifth intercostal space, and the heart is suspended in a pericardial cradle. A 1- to 2-cm segment of the left circumflex coronary artery (LCX) is isolated proximal to the first major diagonal ventricular branch. A 26-gauge needle-tipped wire anodal electrode (Teflon-coated, 30-gauge silverplated copper wire) 3-4 mm long is inserted into the LCX and placed in contact with the intimal surface of the artery (confirmed at the end of the experiment). The stimulating circuit is completed by placing the cathode in a subcutaneous (s.c.) site. An adjustable plastic occluder is placed around the LCX, over the region of the electrode. A precalibrated electromagnetic flow probe (Carolina Medical Electronics, King, NC, U.S.A.) is placed around the LCX proximal to the anode for measurement of coronary blood flow (CBF). The occluder is adjusted to produce a 40-50 percent inhibition of the hyperemic blood flow response observed after 10-s mechanical occlusion of the LCX. All hemodynamic and ECG measurements are recorded and analyzed with a data acquisition system (model M3000, Modular Instruments, Malvern, PA. U.S.A.).

Thrombus Formation and Compound Administration Regimens

Electrolytic injury of the intima of the LCX is produced by applying 100-μA direct current (DC) to the anode. The current is maintained for 60 min and then discontinued whether the vessel has occluded or not. Thrombus formation proceeds spontaneously until the LCX is totally occluded (determined as zero CBF and an increase in the S-T segment).

Compound administration is started after the occluding thrombus is allowed to age for 1 hour. A 2-hour infusion of the compounds of the present invention at doses of 0.5 and 1 mg/kg/hour is begun simultaneously with an infusion of thrombolytic agent (e.g. tissue plasminogen activator, streptokinase, APSAC). Reperfusion is followed for 3 hour after administration of test compound. Reocclusion of coronary arteries after successful thrombolysis is defined as zero CBF which persisted for at least 30 minutes.

Hematology and template bleeding time determinations

Whole blood cell counts, hemoglobin, and hematocrit values are determined on a 40-µL sample of citrated (3.8 percent) blood (1 part citrate:9 parts blood) with a hematology analyzer (Cell-Dyn 900, Sequoia-Turner. Mount View, CA, U.S.A.). Gingival template bleeding times are determined with a Simplate II bleeding time device (Organon Teknika Durham, N.C., U.S.A.). The device is used to make 2 horizontal incisions in the gingiva of either the upper or lower left jaw of the dog. Each incision is 3 mm wide x 2 mm deep. The incisions are made, and a stopwatch is used to determine how long bleeding occurs. A cotton swab is used to soak up the blood as it oozes from the incision. Template bleeding time is the time from incision to stoppage of bleeding. Bleeding times are taken just before administration of test compound (0 min), 60 min into infusion, at conclusion of administration of the test compound (120 min), and at the end of the experiment.

All data are analyzed by one-way analysis of variance (ANOVA) followed by Student-Neuman-Kuels post hoc *t* test to determine the level of significance. Repeated-measures ANOVA are used to determine significant differences between time points during the experiments. Values are determined to be statistically different at least at the level of $p<0.05$. All values are mean ± SEM. All studies are conducted in accordance with the guiding principles of the American Physiological Society. Further details regarding the procedures are described in Jackson, et al., J. Cardiovasc. Pharmacol., (1993), 21, 587-599.

The following Examples are provided to further describe the invention and are not to be construed as limitations thereof.

The abbreviations, symbols and terms used in the examples have the following meanings.

Ac = acetyl
aq = aqueous
Bn or Bzl = benzyl
Boc = t-butyloxycarbonyl
Bu = butyl
n-BuLi = butyllithium
Calcd = calculated
conc = concentrated
DMF = dimethylformamide
DMSO = dimethylsulfoxide
eq = (molar) equivalent
Et = ethyl
EtOAc = ethyl acetate
$Et_3N$ = triethylamine
$Et_2O$ = diethyl ether
EtOH = ethanol
FTIR = Fourier transform IR
Hex = hexanes
HPLC = High Performance Liquid Chromatography
HRMS = high resolution mass spectrum
i-PrOH = isopropanol
IR = Infrared Spectrum
LC-MS = liquid chromatography - mass spectrum (using HPLC)
Me = methyl
MeOH = methanol
MS-ES (or ES-MS) = electrospray mass spectrum
MS-FAB (or FAB-MS) = fast atom bombardment mass spectrum
MS-FIA (or FIA-MS) = flow injection analysis mass spectrum
MS-FD (or FD-MS) = field desorption mass spectrum
MS-IS (or IS-MS) = ion spray mass spectrum
NMR = Nuclear Magnetic Resonance

Ph = phenyl
i-Pr = isopropyl
RPHPLC = Reversed Phase High Performance Liquid Chromatography
RT (or $R_t$) = retention time
satd = saturated
$SiO_2$ = silica gel
SCX = strong cation exchange (resin)
TBS = tert-butyldimethylsilyl
TFA = trifluoroacetic acid
THF = tetrahydrofuran
TIPS = triisopropylsilyl
TLC = thin layer chromatography
tosyl = p-toluenesulfonyl
triflic acid = trifluoromethanesulfonic acid

Unless otherwise stated, pH adjustments and work up are with aqueous acid or base solutions. $^1$H-NMR indicates a satisfactory NMR spectrum was obtained for the compound described. IR (or FTIR) indicates a satisfactory infra red spectrum was obtained for the compound described.

For consistency and clarity, a number of compounds are named as substituted pyridinecarboxamide or pyrazine-carboxamide derivatives.

**Example 1**

**Preparation of N-(6-Chloropyridin-3-yl)-2-[[1-(4-pyridinyl)piperidin-4-ylmethyl]amino]pyridine-3-carboxamide Dihydrochloride**

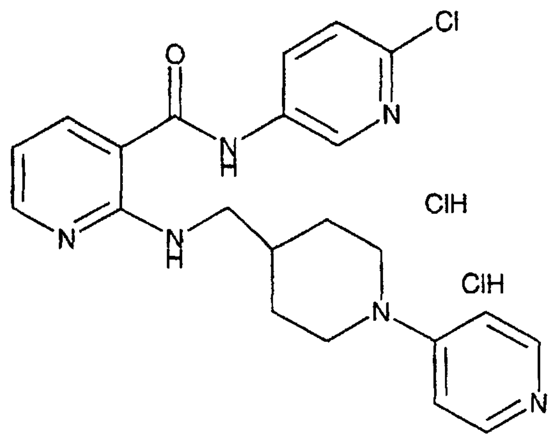

A. 1-(4-Pyridinyl)piperidine-4-methylamine.

1-(4-Pyridinyl)piperidine-4-methanol was prepared using a procedure similar to the following: A solution of methyl N-(4-pyridinyl)isonipecotate (600 mg, 2.72 mmol) in tetrahydrofuran was added to a solution of lithium aluminum hydride (100 mg) in tetrahydrofuran (14 mL) cooled to 0 °C. Upon consumption of the starting material (0.5-2 h), the mixture was treated with water (0.10 mL), 15% aqueous sodium hydroxide (0.10 mL), and water (0.30 mL). After 0.25 h, the mixture was sonicated for 0.25 h, then poured into a mixture of ethyl acetate, water, sodium tartrate, and potassium tartrate. The aqueous layer was extracted twice with ethyl acetate and the combined extracts were dried (magnesium sulfate), filtered, and concentrated in vacuo to yield 357 mg (68%) of 1-(4-pyridinyl)piperidine-4-methanol, which was used without further purification.
$^1$H-NMR

A solution of 1-(4-pyridinyl)piperidine-4-methanol (5.87 g, 30.6 mmol), phthalimide (4.59 g, 31.2 mmol), and triphenylphosphine (8.10 g, 30.9 mmol) in 125 mL of THF at -5 °C was treated with a solution of diethyl azodicarboxylate

(5.38 g, 30.9 mmol) in THF (40 mL). After 16 h, the mixture was poured into EtOAc and 1 N HCl. The aqueous layer was washed with EtOAc (2x), pH adjusted to 12 by addition of 5 N NaOH, and washed with EtOAc (3x). The combined organic extracts were dried ($K_2CO_3$) and concentrated yielding 8.45 g (86%). The crude material (5.47 g, 17.0 mmol) was then treated with hydrazine hydrate (3.5 mL, 60.0 mmol) in EtOH (50 mL). The mixture was heated at 75 °C for 5 h, cooled, diluted with $CH_2Cl_2$ (100 mL), and cooled to 0 °C. The solid was removed by filtration and the filtrate was concentrated yielding 3.32 g of the title compound which was used without further purification.
$^1$H-NMR, IR
FD-MS, m/e 191 (m)

B. Ammonium 2-[[1-(4-Pyridinyl)piperidin-4-ylmethyl]amino]pyridine-3-carboxylate.

A mixture of 2-chloronicotinic acid (10.74 g, 67.5 mmol), 1-(4-pyridinyl)piperidin-4-methylamine (8.60 g, 45.0 mmol), and potassium carbonate (15.5 g, 112.6 mmol) in dimethylformamide (90 mL) was heated at reflux. After 16 h, the mixture was diluted with methanol, filtered, and concentrated. The residue was dissolved in methanol, acidified with 1 N HCl in ether, heated at reflux for 0.25 h, cooled and the solid removed by filtration. The filtrate was then treated with 2 M $NH_3$ in methanol until slightly basic, triturated with THF and the resulting solid collected by filtration yielding 10.75 g of the title compound; which was used without further purification.
$^1$H-NMR
IS-MS, m/e 313 (m+1)

C. N-(6-Chloropyridin-3-yl)-2-[[1-(4-pyridinyl)piperidin-4-ylmethyl]amino]pyridine-3-carboxamide.

A solution of ammonium 2-[[1-(4-pyridinyl)piperidin-4-ylmethyl]amino]pyridine-3-carboxylate (3.0 g, 9.12 mmol) in dioxane (45 mL) was treated with phosgene (1.9 M in toluene, 9.50 mL, 18.2 mmol), and the resulting mixture was heated at reflux. After 2 h, the mixture was concentrated yielding the corresponding aza-isatoic anhydride which was used without further purification. A solution of the crude anhydride (300 mg, 0.648 mmol) in tetrahydrofuran (2 mL) at 0 °C was treated with the magnesium salt of 2-amino-5-chloropyridine [2.60 mmol; freshly prepared by addition of methyl magnesium bromide (3.0 M in THF, 0.865 mL, 2.60 mmol) to 2-amino-5-chloropyridine (900 mg, 3.89 mmol) in THF (10 mL) at 0 °C]. After 17 h, the mixture was treated with a saturated aqueous solution of ammonium chloride and then partitioned between EtOAc and water. The aqueous layer was washed with EtOAc (3x) and the combined extracts were washed with water (1x), dried with sodium sulfate, and concentrated. The residue was purified by RPHPLC yielding 29 mg (9%) of the title compound as a hydrochloride salt.
$^1$H-NMR
IS-MS, m/e (m)

| Analysis for $C_{22}H_{23}ClN_6O \cdot 2.0\ HCl \cdot 1.8\ H_2O$: | | | |
|---|---|---|---|
| Calcd | C, 50.22; | H, 5.10; | N, 15.97; |
| Found | C, 50.48; | H, 5.10; | N, 15.67. |

**Example 2**

**Preparation of N-(5-Chloropyridin-2-yl)-3-[[1-(4-pyridinyl)piperidin-4-ylcarbonyl]amino]pyridine-2-carboxamide Hydrochloride.**

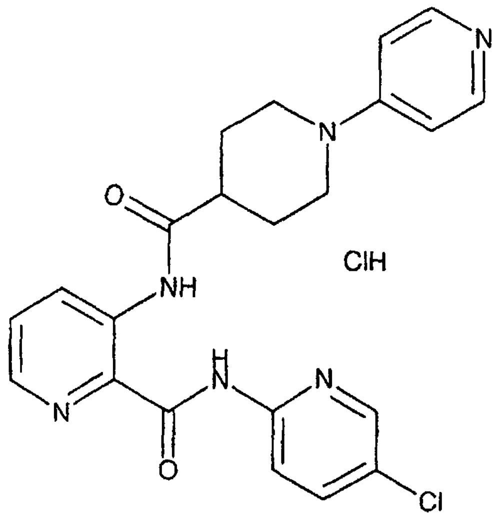

A. 3-Aminopyridine-N-(5-chloropyridin-2-yl)-2-carboxamide.

A (Parr) pressure apparatus was charged with 3-amino-2-chloropyridine (500 mg, 3.89 mmol), 2-amino-5-chloropyridine (1.00 g, 7.78 mmol), palladium acetate (88 mg, 0.39 mmol), 1,3-bis(diphenylphosphino)propane (483 mg, 1.17 mmol) and triethylamine (590 mg, 5.84 mmol). The mixture was placed under a carbon monoxide atmosphere (4.1 bar) and heated at 100 °C. After 72 h, the mixture was filtered, concentrated and the residue purified by column chromatography ($SiO_2$: 0 to 5% EtOAc in methylene chloride) affording 550 mg (57%) of the title compound.
$^1$H-NMR, IR
IS-MS, m/e 249 (m)

| Analysis for $C_{11}H_9ClN_4O$: | | | |
|---|---|---|---|
| Calcd | C, 53.13; | H, 3.65; | N, 22.53; |
| Found | C, 53.40; | H, 3.66; | N, 22.45. |

B. N-(5-Chloropyridin-2-yl)-3-[[1-(4-pyridinyl)piperidin-4-ylcarbonyl]amino]pyridine-2-carboxamide hydrochloride.

3-Aminopyridine-N-(5-chloropyridin-2-yl)-2-carboxamide (5.73 g, 23.0 mmol) was added to a solution of pyridine (3.92 mL) and 1-(4-pyridinyl)piperidin-4-yl carbonyl chloride (24.2 mmol, prepared by addition of oxalyl chloride [26.7 mmol] to sodium 1-(4-pyridinyl)piperidine-4-carboxylate [24.2 mmol]) in methylene chloride. After 16 h, the mixture was partitioned between saturated sodium bicarbonate and methylene chloride. The organic layer was dried with magnesium sulfate, filtered, concentrated, and the residue purified by column chromatography ($SiO_2$; 5 to 10% methanol: methylene chloride) followed by recrystallization from EtOAc:hexanes and treatment with hydrochloric acid to yield 842 mg (8%) of the title compound.
$^1$H-NMR, IR

| Analysis for $C_{22}H_{21}ClN_6O_2 \cdot 1.75$ HCl: | | | |
|---|---|---|---|
| Calcd | C, 52.77; | H, 4.57; | N, 16.78; |
| Found | C, 52.93; | H, 4.63; | N, 16.74. |

**Example 3**

**Preparation of 3-[(4-tert-Butylbenzoyl)amino]-N-(5-chloropyridin-2-yl)pyrazine-2-carboxamide.**

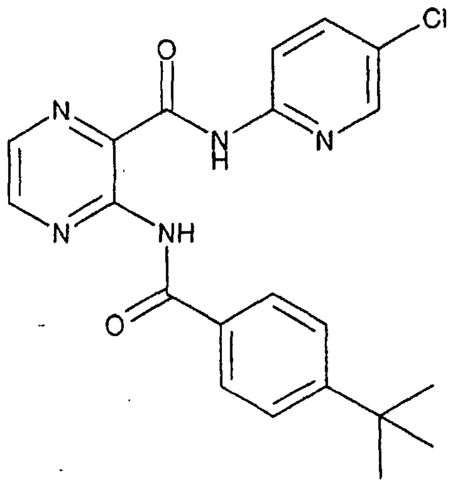

A. 3-Amino-N-(5-chloropyridin-2-yl)pyrazine-2-carboxamide.

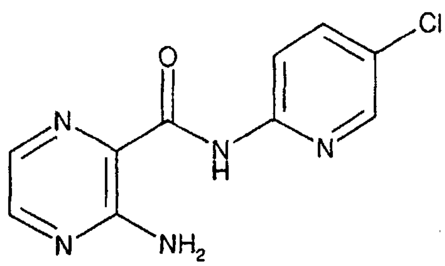

To a cold (0 °C) solution of 3-aminopyrazine-2-carboxylic acid (10 g, 72 mmol) in $CH_2Cl_2$ (250 mL) was added a 2 M solution of oxalyl chloride (43 mL, 86 mmol) in $CH_2Cl_2$ followed by DMF (10 mL) dropwise. The cooling bath was removed, and the reaction was stirred for 2 hours at ambient temperature. It was recooled to 0 °C and treated with a 0 °C solution of 2-amino-5-chloropyridine (12 g, 94 mmol) and pyridine (32 mL) in $CH_2Cl_2$ (200 mL). The cooling bath was removed and the reaction was stirred overnight at ambient temperature. The mixture was concentrated to dryness in vacuo and the residue was mixed with MeOH. The solid was filtered to recover 15.6 g (87%) of product.
$^1$H-NMR
MS, m/e 249 (m)

| Analysis for $C_{10}H_8N_5O \cdot 0.3H_2O$: | | | |
|---|---|---|---|
| Calcd | C, 47.09; | H, 3.40; | N, 27.46; |
| Found | C, 47.38; | H, 3.24; | N, 27.14. |

B. 3-[(4-tert-Butylbenzoyl)amino]-N-(5-chloropyridin-2-yl)pyrazine-2-carboxamide.

To a mixture of the above 3-amino-N-(5-chloropyridin-2-yl)pyrazine-2-carboxamide (255 mg, 1 mmol) in pyridine (20 mL) was added 4-tert-butylbenzoyl chloride (402 mg, 86 mmol). The mixture was stirred for 36 hours at 55 °C. It was cooled to room temperature then concentrated to dryness in vacuo.- The residue was mixed with $CH_2Cl_2$

and purified by radial chromatography from which was recovered 85 mg (21%) of product.
$^1$H-NMR
MS, m/e 410 (m+1)

| Analysis for $C_{21}H_{20}ClN_5O_2$: | | | |
|---|---|---|---|
| Calcd | C, 61.54; | H, 4.92; | N, 17.09; |
| Found | C, 61.84; | H, 5.09; | N, 17.32. |

**Example 4**

**Preparation of N-(6-Chlorobenzothiazol-2-yl)-2-[[1-(4-pyridinyl)piperidin-4-ylmethyl]amino]pyridine-3-carboxamide.**

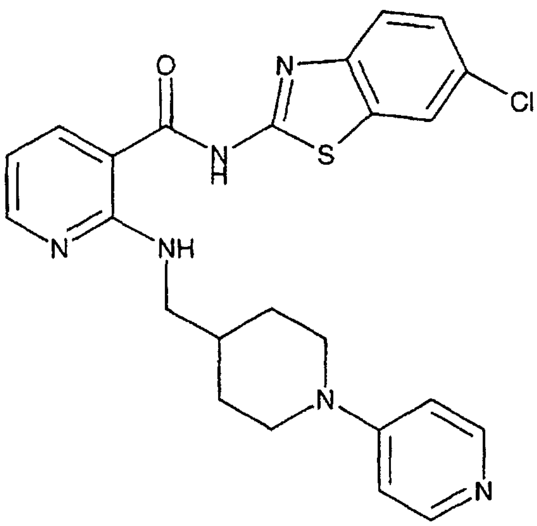

A solution of ammonium 2-[[1-(4-pyridinyl)piperidin-4-ylmethyl]amino]pyridine-3-carboxylate (400 mg, 1.28 mmol) in methylene chloride (5 mL) was treated with thionyl chloride (0.112 mL, 1.54 mmol) and the mixture was heated at reflux. After 3 h, the mixture was cooled and then treated dropwise with a solution of 2-amino-6-chlorobenzothiazole (284 mg, 1.54 mmol) in pyridine (5 mL). After 1 h, the mixture was concentrated in the presence of silica gel. The residue was purified by column chromatography ($SiO_2$: 1 to 4% [2 N ammonia in methanol]:chloroform) followed by recrystallization from methanol to yield 68 mg (11%) of the title compound.
$^1$H-NMR
IS-MS, m/e 480 (m+1)

| Analysis for $C_{24}H_{23}ClN_6OS$: | | | |
|---|---|---|---|
| Calcd | C, 60.18; | H, 4.84; | N, 17.54; |
| Found | C, 60.41; | H, 4.98; | N, 17.56. |

**Example 5**

**Preparation of N-(5-Chloropyridin-2-yl)-2-[[1-(4-pyridinyl)-piperidin-4-ylcarbonyl]amino]pyridine-3-carboxamide Hydrochloride.**

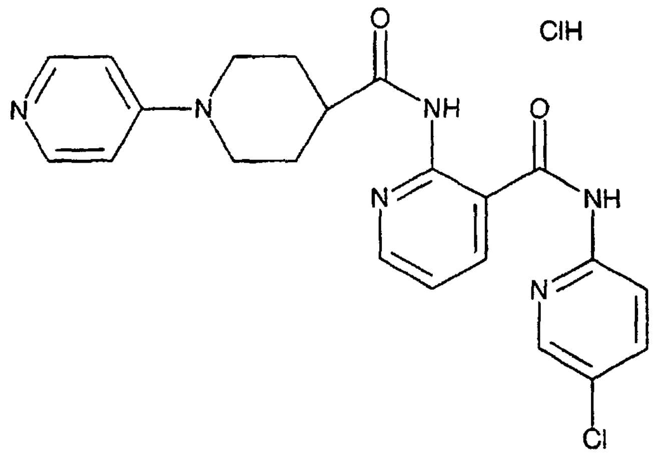

A. 2-Amino-N-(5-chloropyridin-2-yl)pyridine-3-carboxamide hydrochloride.

To a stirring suspension of 2-aminonicotinic acid (26.9 g, 194 mmol) in dichloromethane (120 mL) at 0 °C, was added DMF (a few drops) followed by oxalyl chloride (20 mL, 194 mmol). The cold bath was removed and the solution was allowed to stir for 60 min at room temperature. This solution was then transferred via cannula into a stirring solution of 2-amino-5-chloropyridine (25 g, 194 mmol) and pyridine (78 mL, 970 mmol) in dichloromethane (100 mL). After stirring overnight, the precipitate was filtered and dried to give 32.8 g of solid. The crude product was recrystalized from ethanol with activated charcoal to give 12.4 g (23 %) of white solid.
$^1$H-NMR
IS-MS, m/e 249.0 (m+1)

| Analysis for $C_{11}H_9N_4OCl \cdot HCl$: | | | | |
|---|---|---|---|---|
| Calcd | C, 46.33; | H, 3.54; | N, 19.65; | Cl, 24.87; |
| Found | C, 46.64; | H, 3.42; | N, 19.63; | Cl, 25.23. |

B. N-(5-Chloropyridin-2-yl)-2-[[1-(4-pyridinyl)piperidin-4-ylcarbonyl]amino]pyridine-3-carboxamide hydrochloride.

By methods substantially equivalent to those described in example 2-B, N-(5-chloropyridin-2-yl)-2-[[1-(4-pyridinyl)piperidin-4-ylcarbonyl]amino]pyridine-3-carboxamide hydrochloride (84 mg, 4 %) was prepared from 2-amino-N-(5-chloropyridin-2-yl)pyridine-3-carboxamide and 1-(4-pyridinyl)piperidine-4-carbonyl chloride. The compound was purified by preparative RPHPLC (C18), eluting with a linear gradient of 90/10 to 50/50 (0.01% HCl/acetonitrile) over 180 min.
$^1$H-NMR
IS-MS, m/e 437.2 (m+1)

**Example 6**

**Preparation of N-(5-Chloropyridin-2-yl)-3-[(1-isopropylpiperidin-4-ylcarbonyl)amino]pyridine-2-carboxamide Hydrochloride.**

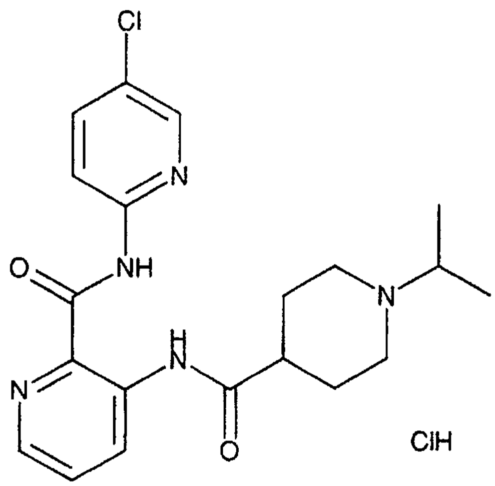

A. N-(5-Chloropyridin-2-yl)-3-[(1-Boc-piperidin-4-yl-carbonyl)amino]pyridine-2-carboxamide.

To a stirring solution of Boc-isonipecotic acid (15.4 g, 67 mmol) in THF (200 mL) was added sodium methoxide (3.62 g, 67 mmol). After stirring for 1 h, the solvent was removed in vacuo and the dry residue was suspended in dichloromethane (100 mL). To this stirring suspension was added a few drops of DMF followed by oxalyl chloride (6.5 mL, 74 mmol). After stirring for 2 h, the solvents were removed in vacuo and the residue was diluted to a volume of about 130 mL with dichloromethane.

A portion of this solution (81 mL, 40 mmol) was added via syringe to a stirring solution of N-(5-chloropyridin-2-yl)-3-aminopyridine-2-carboxamide (6.7 g, 26.9 mmol), 4-dimethylaminopyridine (0.49 g, 4 mmol) and N,N-diisopropylethylamine (8.2 mL, 47.1 mmol) in dichlqromethane (200 mL). After stirring overnight, the solvent was removed in vacuo and the residue was partitioned between ethyl acetate and water. The phases were separated and the organic phase was washed consecutively with 0.5 M citric acid (2X), brine, sat. aq. sodium bicarbonate (2X), and brine. The solution was then dried with $MgSO_4$, filtered, concentrated in vacuo, and the residue was chromatographed over silica gel, eluting with dichloromethane, followed by 10% ethyl acetate in dichloromethane. The product containing fractions were combined and concentrated in vacuo to give 8.69 g (72%) of a white solid.
$^1$H-NMR
IS-MS, m/e 460.4 (m+1)

B. N-(5-Chloropyridin-2-yl)-3-[(piperidin-4-ylcarbonyl)amino]pyridine-2-carboxamide Trifluoroacetate.

To a stirring solution of N-(5-chloropyridin-2-yl)-3-[(1-Boc-piperidin-4-ylcarbonyl)amino]pyridine-2-carboxamide (8.5 g, 18.5 mmol) and anisole (20 mL) in dichloromethane (150 mL) was added TFA (150 mL). After stirring for 1 h, the solvent was removed in vacuo and the residue was suspended in diethyl ether with vigorous stirring. After 30 min, the solid was filtered, washed several times with diethyl ether and then dried in vacuo to give 8.53 g (97%) of a white solid.
$^1$H-NMR
IS-MS, m/e 360.2 (m+1)

| Analysis for $C_{17}H_{18}ClN_5O_2$: | | | | |
|---|---|---|---|---|
| Calcd | C, 47.52; | H, 3.97; | N, 14.43; | F, 12.92; |
| Found | C, 47.81; | H, 3.98; | N, 14.36; | F, 12.76. |

C. N-(5-Chloropyridin-2-yl)-3-[(1-isopropylpiperidin-4-yl-carbonyl)amino]pyridine-2-carboxamide Hydrochloride.

To a stirring suspension of N-(5-chloropyridin-2-yl)-3-[(piperidin-4-ylcarbonyl)amino]pyridine-2-carboxamide trifluoroacetate (0.34 g, 0.72 mmol) in 1,2-dichloroethane (10 mL) was added acetone (10 mL), followed by acetic acid (0.7 mL, 2.88 mmol) and then sodium triacetoxyborohydride (0.61 g, 2.88 mmol). After stirring overnight, the solution was loaded onto an SCX column (prewashed with 5% acetic acid in methanol) and washed with methanol. The product was then eluted from the column with a 2 N solution of ammonia in methanol. The product containing fractions were concentrated in vacuo and the compound was purified by preparative RPHPLC (C18), eluting with a linear gradient of 90/10 to 50/50 (0.01% HCl/acetonitrile) over 180 min, to give 0.14 g (44%) of a white solid.
$^1$H-NMR
IS-MS, m/e 402.3 (m+1)

| Analysis for $C_{20}H_{24}N_5O_2Cl\cdot1.0HCl\cdot1.5H_2O$ : | | | | |
|---|---|---|---|---|
| Calcd | C, 51.62; | H, 6.06; | N, 15.05; | Cl, 15.24; |
| Found | C, 51.61; | H, 5.75; | N, 14.80; | Cl, 15.31. |

**Example 7**

**Preparation of N-(5-Methylpyridin-2-yl)-2-[[1-(4-pyridinyl)piperidin-4-ylmethyl]amino]pyridine-3-carboxamide Dihydrochloride.**

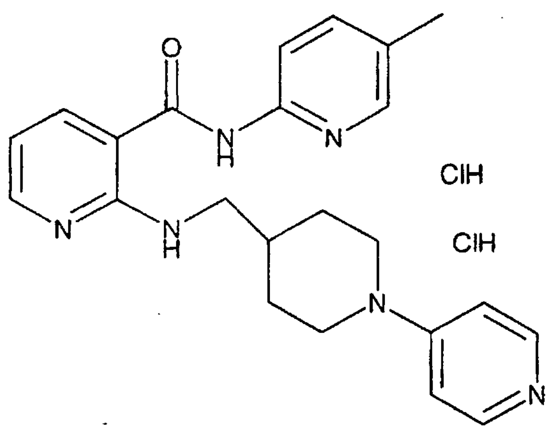

Using a similar procedure to that described in Example 1-C, the crude anhydride (300 mg, 0.648 mmol) and 2-amino-5-methylpyridine (280 mg, 2.60 mmol) yielded 64 mg (20%) of the title compound as a hydrochloride salt.
$^1$H-NMR, IR
IS-MS, m/e 403 (m+1)

| Analysis for $C_{23}H_{26}N_6O\cdot2.0$ HCl: | | | |
|---|---|---|---|
| Calcd | C, 58.11; | H, 5.94; | N, 17.68; |
| Found | C, 58.35; | H, 5.85; | N, 17.60. |

**Example 8**

**Preparation of N-(6-Chloropyridazin-3-yl)-2-[[1-(4-pyridinyl)piperidin-4-ylmethyl]amino]pyridine-3-carboxamide Dihydrochloride.**

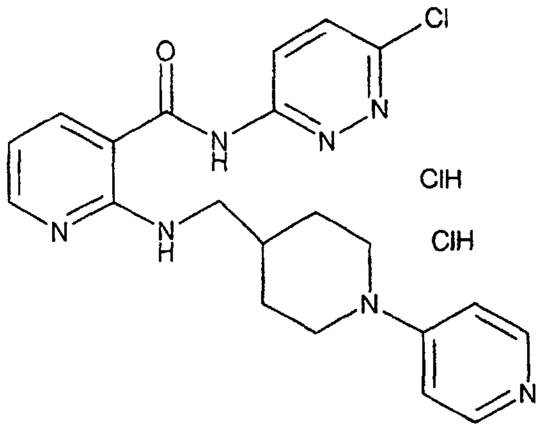

Using a similar procedure to that described in Example 1-C, the crude anhydride (300 mg, 0.648 mmol) and 3-amino-5-chloropyridazine (335 mg, 2.60 mmol) yielded 46 mg (14%) of the title compound as a hydrochloride salt.
$^{1}$H-NMR
IS-MS, m/e 424 (m+1)

**Example 9**

**Preparation of N-(Benzothiazol-2-yl)-2-[[1-(4-pyridinyl)piperidin-4-ylmethyl]amino]pyridine-3-carboxamide Dihydrochloride.**

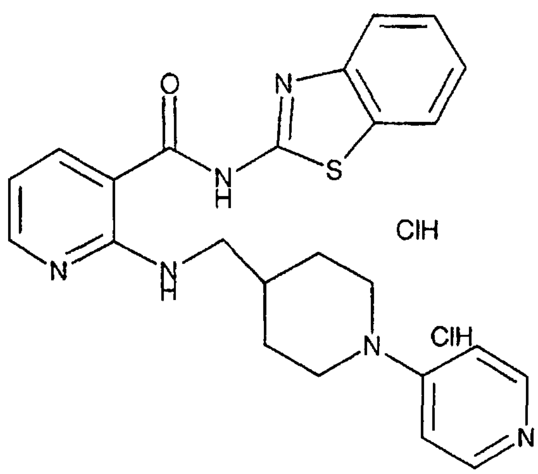

Using a similar procedure to that described in Example 1-C, the crude anhydride (300 mg, 0.648 mmol) and 2-amino-benzothiazole (390 mg, 2.60 mmol) yielded 70 mg (24%) of the title compound as a hydrochloride salt.
$^{1}$H-NMR
IS-MS, m/e 445 (m+1)

| Analysis for $C_{24}H_{24}N_6OS \cdot 3.0\ HCl,\ 1.5\ H_2O$: | | | |
|---|---|---|---|
| Calcd | C, 49.78; | H, 5.19; | N, 14.51; |
| Found | C, 49.63; | H, 4.76; | N, 14.22. |

**Example 10**

**Preparation of N-(6-Methylbenzothiazol-2-yl)-2-[[1-(4-pyridinyl)piperidin-4-ylmethyl)amino]pyridine-3-carboxamide Dihydrochloride.**

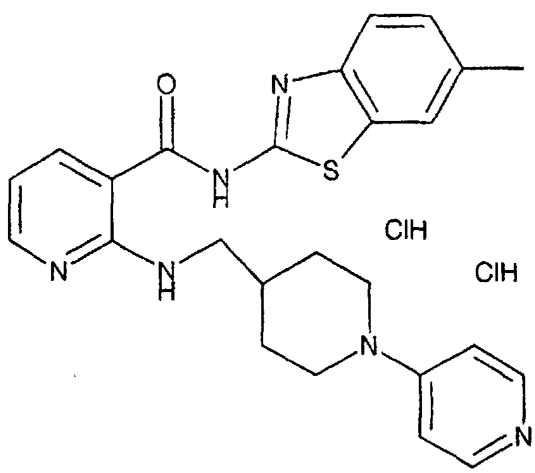

Using a similar procedure to that described in Example 1-C, the crude anhydride (300 mg, 0.648 mmol) and 2-amino-6-methylbenzothiazole (426 mg, 2.60 mmol) yielded 66 mg (19%) of the title compound as a hydrochloride salt.
$^1$H-NMR
IS-MS, m/e 445 (m+1)

| Analysis for $C_{25}H_{26}N_6OS \cdot 3.0\ HCl \cdot 1.0\ H_2O$: | | | |
|---|---|---|---|
| Calcd | C, 51.25; | H, 5.33; | N, 14.34; |
| Found | C, 51.51; | H, 5.14; | N, 14.28. |

**Example 11**

**Preparation of N-(6-Bromobenzothiazol-2-yl)-2-[[1-(4-pyridinyl)piperidin-4-ylmethyl]amino]pyridine-3-carboxamide Dihydrochloride.**

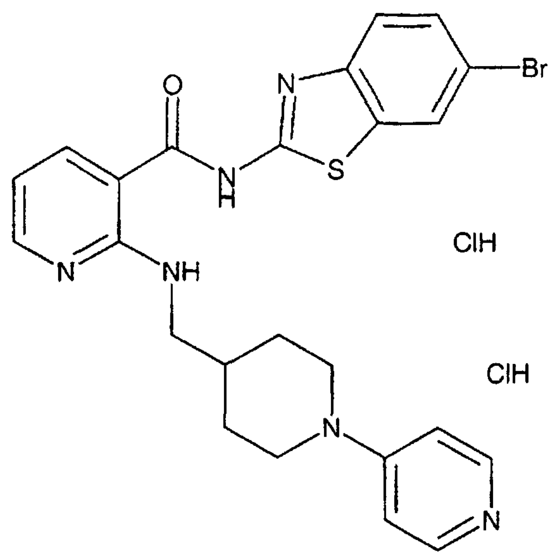

Using a similar procedure to that described in Example 1-C, the crude anhydride (300 mg, 0.648 mmol) and 2-amino-6-bromobenzothiazole (595 mg, 2.60 mmol) yielded 8.0 mg (2%) of the title compound as a hydrochloride salt.
$^1$H-NMR
IS-MS, m/e 524 (m+1)

**Example 12**

**Preparation of N-(5-Chloropyrimindin-2-yl)-2-[[1-(4-pyridinyl)piperidin-4-ylmethyl]amino]pyridine-3-carboxamide Dihydrochloride.**

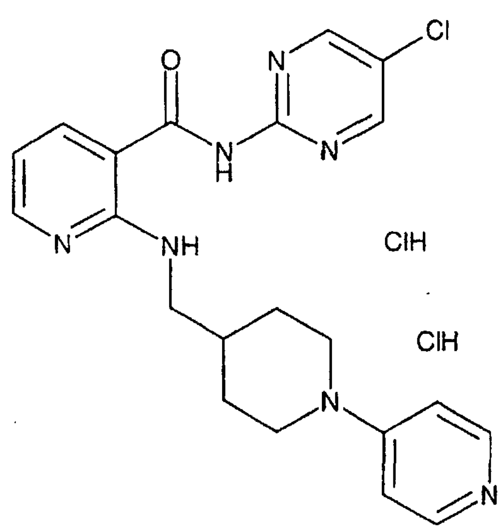

Using a similar procedure to that described in Example 1-C, the crude anhydride (300 mg, 0.648 mmol) and 2-amino-5-chloropyrimidine (595 mg, 2.60 mmol) yielded 8.0 mg (2%) of the title compound as a hydrochloride salt.
$^1$H-NMR
IS-MS, m/e 524 (m+1)

**Example 13**

**Preparation of N-(5-Chloropyridin-2-yl)-2-[(1-isopropylpiperidin-4-ylmethyl)amino]pyridine-2-carboxamide.**

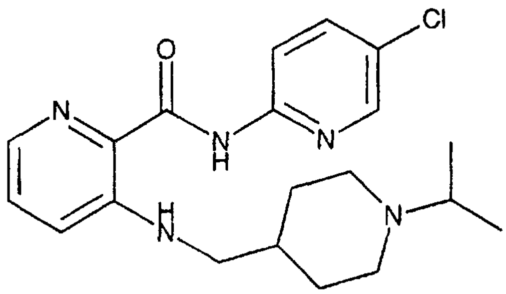

A. 1-*tert*-Butoxycarbonylpiperidine-4-methanol.

A solution of 1-*tert*-butoxycarbonyl isonipecotic acid (40 g, 0.17 mol) and N-methylmorpholine (19 mL, 0.17 mol) in tetrahydrofuran (900 mL) at -10 °C was treated with ethyl chloroformate (17 mL, 0.17 mol). After 0.5 h, sodium borohydride was added (19.8 g, 0.5 mol) in one portion followed by slow addition of methanol. After gas evolution ceased, the mixture was concentrated and the residue was diluted with 10% aqueous acetic acid and partitioned between ethyl acetate and water. The aqueous layer was washed with EtOAc'(2x) and the combined organic extracts were dried with magnesium sulfate, filtered, and concentrated to a solid residue which was purified by column chromatography ($SiO_2$: 10 to 50% EtOAc:hexanes) providing the title compound (33.8 g, 90%) as a white solid.
$^1$H-NMR

B. 1-*tert*-Butoxycarbonylpiperidine-4-carboxaldehyde.

A solution of oxalyl chloride (6 mL, 70 mmol) in dichloromethane (60 mL) at -78 °C was treated dropwise with dimethyl sulfoxide (10 mL, 0.14 mol). After 15 minutes, 1-*tert*-butoxycarbonylpiperidine-4-methanol (3.0 g, 14 mmol) was added as a solution in dichloromethane (35 mL). The mixture was stirred at -78 °C for 1 h, then triethylamine (29 mL, 0.21 mol) was added dropwise. The mixture was warmed to ambient temperature and poured into saturated ammonium chloride solution (200 mL). The organic layer was separated and the aqueous layer was washed with dichloromethane (75 mL). The organic layers were combined and washed with brine (75 mL), then dried with $MgSO_4$, filtered and concentrated. The residue was redissolved in ethyl acetate-hexanes (1:1) and filtered through Florisil (100-200 mesh). The resulting filtrate was concentrated yielding 3.0 g (100%) of the title aldehyde as a yellow oil; which was used without further purification.
$^1$H-NMR.

C. N-(5-Chloropyridin-2-yl)-3-[(1-Boc-piperidin-4-yl-methylidine)amino]pyridine-2-carboxamide.

A solution containing 1-*tert*-butoxycarbonylpiperidine-4-carboxaldehyde (2.00 g, 9.38 mmol), 3-amino-N-(5-chloropyridin-2-yl)pyridine-2-carboxamide (2.33 g, 9.38 mmol), and pyridinium p-toluenesulfonate (236 mg, 0.94 mmol) in benzene (100 mL) was heated at reflux with azeotropic removal of water. After 48 h, the mixture was concentrated and the residue was purified by column chromatography ($SiO_2$: methylene chloride) yielding the title compound, which was contaminated with 3-amino-N-(5-chloropyridin-2-yl)pyridine-2-carboxamide and was used without further purification.
$^1$H-NMR
IS-MS, m/e 444(m)

D. N-(5-Chloropyridin-2-yl)-3-[(piperidin-4-ylmethyl)amino]pyridine-2-carboxamide.

A solution containing N-(5-chloropyridin-2-yl)-3-[(1-Boc-piperidin-4-ylmethylidine)amino]pyridine-2-carboxamide (crude obtained from Part C) and borane trimethylamine complex (2.05 g, 28.14 mmol) in glacial acetic acid was heated at reflux for 2 h. The mixture was cooled, concentrated, and the residue was dissolved in methanol (100 mL) and 12 N HCl (10 mL). After 24 h, the mixture was concentrated, the residue was partitioned between EtOAc and water, and the organic layer washed with a saturated potassium carbonate solution. The organic layer was dried with magnesium sulfate, filtered, concentrated, and the residue was purified by column chromatography ($SiO_2$: 10% [2 N ammonia in methanol]:methylene chloride) yielding 1.63 g (50%) of the title compound.
$^1$H-NMR, IR
IS-MS, m/e 346 (m)

| Analysis for $C_{17}H_{20}ClN_5O$: | | | |
|---|---|---|---|
| Calcd | C, 59.04; | H, 5.83; | N, 20.25; |
| Found | C, 58.76; | H, 5.84; | N, 20.05. |

E. N-(5-Chloropyridin-2-yl)-2-[(1-isopropylpiperidin-4-ylmethyl)amino]pyridine-2-carboxamide.

A solution of N-(5-chloropyridin-2-yl)-3-[(piperidin-4-ylmethyl)amino]pyridine-2-carboxamide (100 mg, 0.29 mmol), acetone (1 mL), and anhydrous magnesium sulfate (500 mg) in 95:5 methanol:acetic acid (10 mL) was treated with sodium cyanoborohydride (73 mg, 1.16 mmol). After 4 days, the mixture was filtered, concentrated, and the residue purified by column chromatography ($SiO_2$: 1 to 20% methanol:methylene chloride) yielding 100 mg (89%) of the title compound.
$^1$H-NMR, IR
IS-MS, m/e 388(m+1)

| Analysis for $C_{20}H_{26}ClN_5O \cdot 0.25\ H_2O$: | | | |
|---|---|---|---|
| Calcd | C, 61.22; | H, 6.81; | N, 17.25; |
| Found | C, 61.35; | H, 6.46; | N, 17.20. |

**Example 14**

**Preparation of 5-Chloro-N-(5-chloropyridin-2-yl)-2-[[1-(4-pyridinyl)piperidin-4-ylmethyl]amino]pyridine-3-carboxamide Tetrahydrochloride.**

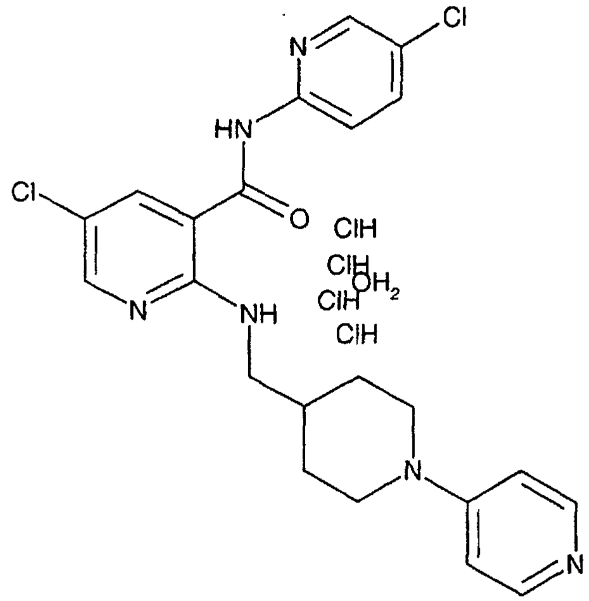

A. N-(5-Chloropyridin-2-yl)-2,5-dichloropyridine-3-carboxamide.

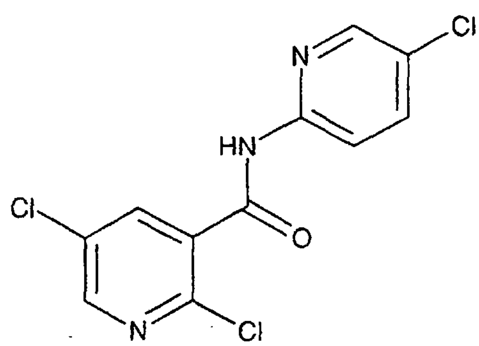

To a ice cooled solution of 2-amino-5-chloropyridine (20 g,95 mmol) in dichloromethane (200 mL) and pyridine (20 mL) was added 2,5-dichloronicotinoyl chloride (11.58 g, 90 mmol)dropwise. The reaction mixture was warmed to room temperature and stirred overnight. The solvent was evaporated and the residue was partitioned between ethyl acetate (500 mL) and water (100 mL). The aqueous layer was extracted with ethyl acetate (3 X 100 mL). Combined organic layers, and washed with water (100 mL), saturated citric acid solution (2 X 100 mL), saturated sodium bicarbonate (2 X 100 mL)and water (200 mL). Dried over magnesium sulfate and evaporated off the solvent. The residue was slurried with ether (100 mL), and the off-white solids were collected by filtration (24 g, 88%).
$^1$H-NMR
FD-MS, m/e 302.1 (m+1)

| Analysis for $C_{11}H_6Cl_3N_3O$: | | | |
|---|---|---|---|
| Calcd | C, 43.67; | H, 2.0; | N, 13.89; |
| Found | C, 43.97; | H, 1.88; | N, 13.97. |

B. 5-Chloro-N-(5-chloropyridin-2-yl)-2-[[1-(4-pyridinyl)piperidin-4-ylmethyl]amino]pyridine-3-carboxamide tetrahydrochloride.

A suspension of N-(5-chloropyridin-2-yl)-2,5-dichloropyridine-3-carboxamide (0.807 g, 2.68 mmol) and 1-(4-pyridinyl)piperidine-4-methylamine (510 mg) in ethyl alcohol (5 mL) was heated in a sealed tube for 24 hours. Filtered the solids and purified by RPHPLC. The pure product containing fractions were combined and lyophilized to give 0.1 g of an off-white solid.
$^1$H-NMR
FD-MS, m/e 457.4 (m+1)

| Analysis for $C_{22}H_{22}Cl_2N_6O \cdot 4HCl \cdot H_2O$: | | | |
|---|---|---|---|
| Calcd | C, 42.54; | H, 4.54; | N, 13.53; |
| Found | C, 42.95; | H, 4.57; | N, 13.58. |

**Example 15**

**Preparation of N-(5-Chloropyridin-2-yl)-3-[[1-(tetrahydropyran-4-yl)piperidin-4-ylcarbonyl]amino]pyridine-2-carboxamide Hydrochloride.**

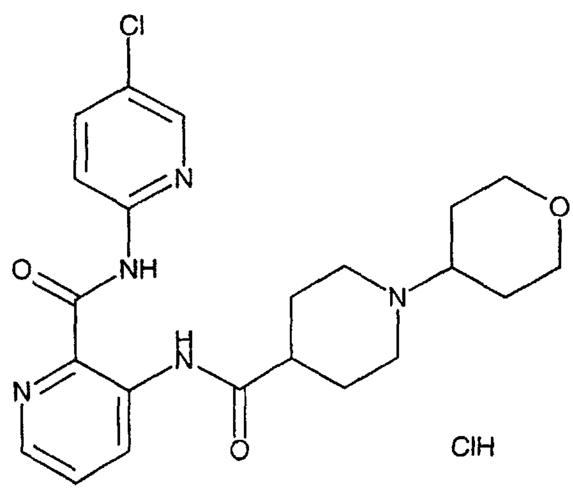

By methods substantially equivalent to those described in example 6-C, N-(5-chloropyridin-2-yl)-3-[[1-(tetrahydropyran-4-yl)piperidin-4-ylcarbonyl]amino]pyridine-2-carboxamide hydrochloride (0.138 g, 34%) was prepared from N-(5-chloropyridin-2-yl)-3-[(piperidin-4-ylcarbonyl)amino]pyridine-2-carboxamide trifluoroacetate and tetrahydropyran-4-one. The compound was purified by preparative RPHPLC (C18)', eluting with a linear gradient of 80/20 to 50/50 (0.01% HCl/acetonitrile) over 180 min.
$^1$H-NMR
IS-MS, m/e 444.2 (m+1)

| Analysis for $C_{22}H_{26}N_5O_3Cl \cdot 1.2HCl \cdot 1.0H_2O$: | | | | |
|---|---|---|---|---|
| Calcd | C, 52.25; | H, 5.82; | N, 13.85; | Cl, 15.42; |
| Found | C, 52.18; | H, 5.48; | N, 13.97; | Cl, 15.27. |

**Example 16**

**Preparation of N-(5-Chloropyridin-2-yl)-2-[(1-cyclopentylpiperidin-4-ylmethyl)amino]pyridine-2-carboxamide.**

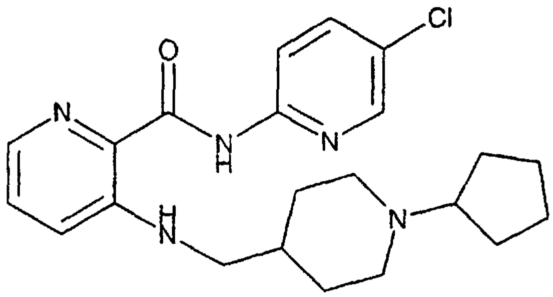

Using a similar procedure to that described in Example 13-E, N-(5-chloropyridin-2-yl)-3-[(piperidin-4-ylmethyl) amino]pyridine-2-carboxamide (100 mg, 0.29 mmol) and cyclopentanone (122 mg, 1.45 mmol) yielded 70 mg (58%) of the title compound.

$^1$H-NMR, IR

IS-MS, m/e 414(m+1)

| Analysis for $C_{22}H_{28}ClN_5O{\cdot}0.5\ H_2O$: | | | |
|---|---|---|---|
| Calcd | C, 62.47; | H, 6.91; | N, 16.56; |
| Found | C, 62.95; | H, 6.69; | N, 16.07. |

**Example 17**

**Preparation of N-(5-Chloropyridin-2-yl)-3-[(1-cyclohexylpiperidin-4-ylcarbonyl)amino]pyridine-2-carboxamide Hydrochloride.**

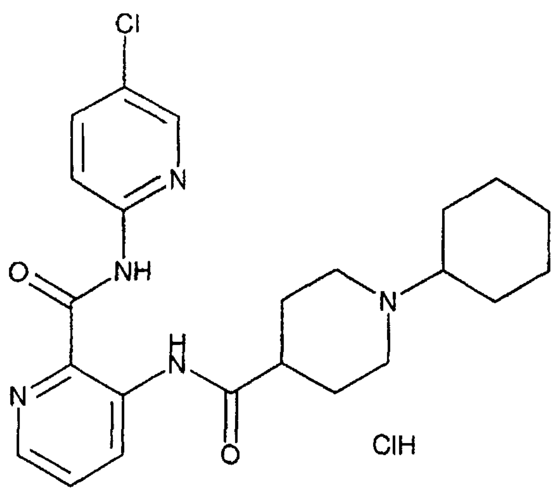

By methods substantially equivalent to those described in example 6-C, N-(5-chloropyridin-2-yl)-3-[(1-cyclohexylpiperidin-4-ylcarbonyl)amino]pyridine-2-carboxamide hydrochloride (0.183 g, 46%) was prepared from N-(5-chloropyridin-2-yl)-3-[(piperidin-4-ylcarbonyl)amino]pyridine-2-carboxamide trifluoroacetate and cyclohexanone.

The compound was purified by preparative RPHPLC (C18), eluting with a linear gradient of 80/20 to 50/50 (0.01% HCl/acetonitrile) over 180 min.
$^1$H-NMR
IS-MS, m/e 442.2 (m+1)

| Analysis for $C_{23}H_{28}N_5O_2Cl \cdot 1.1HCl \cdot 1.0H_2O$: | | | | |
|---|---|---|---|---|
| Calcd | C, 55.24; | H, 6.27; | N, 14.01; | Cl, 14.89; |
| Found | C, 55.04; | H, 6.01; | N, 13.89; | Cl, 14.59. |

**Example 18**

**Preparation of N-(5-Chloropyridin-2-yl)-3-[[1-(4-pyridinyl)piperidin-4-ylmethyl]amino]pyridine-2-carboxamide.**

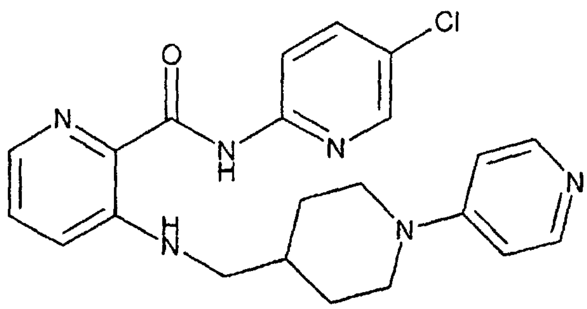

A pressure tube (Aldrich) was charged with N-(5-chloropyridin-2-yl)-3-[(piperidin-4-ylmethyl)amino]pyridine-2-carboxamide (500 mg, 1.45 mmol), 4-chloropyridine hydrochloride (435 mg, 2.90 mmol), triethylamine (293 mg, 2.90 mmol) and EtOH (5 mL), sealed, and placed in a 110 °C bath. After 16 h, the mixture was cooled, concentrated, and the residue purified by column chromatography ($SiO_2$: 5 to 7.5% methanol:methylene chloride) yielding 100 mg (16%) of the title compound.
$^1$H-NMR, IR
IS-MS, m/e 423 (m+1)

| Analysis for $C_{22}H_{23}ClN_6O$: | | | |
|---|---|---|---|
| Calcd | C, 62.48; | H, 5.48; | N, 19.87; |
| Found | C, 61.94; | H, 5.52; | N, 19.71. |

**Example 19**

**Preparation of N-(5-Chloropyridin-2-yl)-3-[(1-isoamylpiperidin-4-ylcarbonyl)amino]pyridine-2-carboxamide Hydrochloride.**

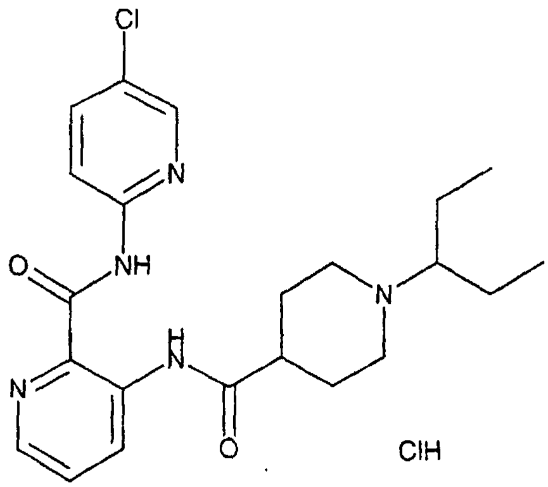

By methods substantially equivalent to those described in example 6-C, N-(5-chloropyridin-2-yl)-3-[(1-isoamylpiperidin-4-ylcarbonyl)amino]pyridine-2-carboxamide hydrochloride (0.083 g, 21%) was prepared from N-(5-chloropyridin-2-yl)-3-[(piperidin-4-ylcarbonyl)amino]pyridine-2-carboxamide trifluoroacetate and 3-pentanone. The compound was purified by preparative RPHPLC (C18), eluting with a linear gradient of 80/20 to 50/50 (0.01% HCl/acetonitrile) over 180 min.

$^1H$-NMR

IS-MS, m/e 430.4 (m+1)

| Analysis for $C_{22}H_{28}N_5O_2Cl \cdot 1.0HCl \cdot 0.2H_2O$: | | | | |
|---|---|---|---|---|
| Calcd | C, 56.22; | H, 6.30; | N, 14.90; | Cl, 15.09; |
| Found | C, 56.22; | H, 6.29; | N, 14.77; | Cl, 15.46. |

**Example 20**

**Preparation of 5-Chloro-N-(5-chloropyridin-2-yl)-2-[(1-isopropylpiperidin-4-ylmethyl)amino]pyridine-3-carboxamide Dihydrochloride.**

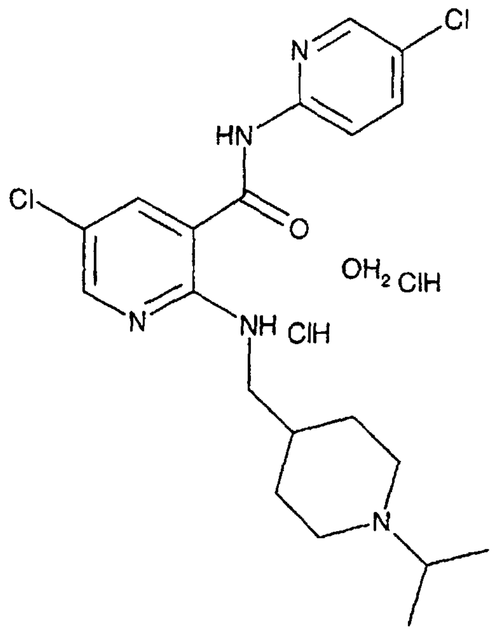

A. 1-Isopropylpiperidine-4-carboxamide.

A solution of 200 mL of DMF, containing 50.0 g of isonicotinamide and 60 mL of 2-bromopropane, was refluxed 5.75 h. A white insoluble solid filtered from this cool solution gave 64.9 g (65%) of 1-isopropylpyridinium-4-carboxamide bromide, m/e= 165, NMR. Catalytic reduction of this salt, with $PtO_2$ in MeOH, gave 65.2 g (98%) of 1-isopropylpiperidine-4-carboxamide hydrobromide, m/e = 171. An aqueous solution of this salt was basified, evaporated to dryness, and extracted with EtOAc to give 39.7 g (90%) of 1-isopropylpiperidine-4-carboxamide free base.

B. 1-Isopropylpiperidine-4-methylamine.

To a suspension of 10.0 g of LAH, in 500 mL of dry THF, at room temperature, was added portionwise 39.7 g of 1-isopropylpiperidine-4-carboxamide, and the mixture was refluxed 18 h. The cooled reaction mixture was diluted with 150 mL THF and treated dropwise with 10 mL $H_2O$ and 10 mL 5 N NaOH, respectively. Gray mixture was refluxed 18 h, filtered and evaporated. Residue partially dissolved in hexane to give 25.5 g of crude yellow liquid and 6.9 g hexane insoluble starting carboxamide. HPLC purification of 25.5 g liquid with 20% MeOH-EtOAc/Silica Gel gave 1-isopropylpiperidine-4-methylamine (8.5 g, 28%).
NMR, m/e = 157.

C. 5-Chloro-N-(5-chloropyridin-2-yl)-2-[(1-isopropylpiperidin-4-ylmethyl)amino]pyridine-3-carboxamide hydrochloride.

A suspension of N-(5-chloropyridin-2-yl)-2,5-dichloropyridine-3-carboxamide 0.607 g, 2.05 mmol) and 1-isopropylpiperidine-4-methylamine (510 mg) in acetonitrile (5 mL) was heated in a sealed tube for 24 hours. Filtered the solids and purified by RPHPLC and the pure product containing fractions were combined and lyophilized to give 0.256 g of tan powder.
$^1$H-NMR
FD-MS, m/e 422.1 (m+1)

**Example 21**

**Preparation of 4-[(4-t-Butylbenzoyl)amino]-N-(5-chloropyridin-2-yl)pyridine-3-carboxamide.**

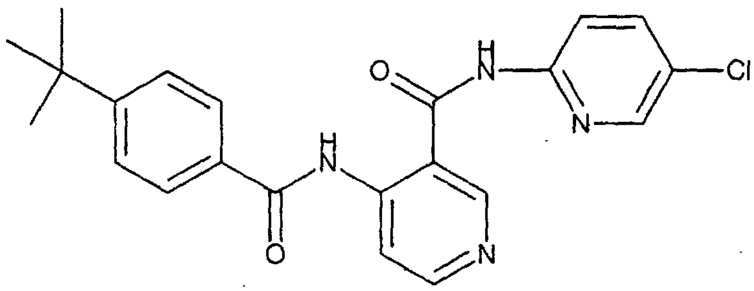

A. 4-(Boc-amino)pyridine.

To a stirring solution of 4-aminopyridine (15 g, 159 mmol) and triethylamine (24 mL, 175 mmol) in DMF (300 mL) was added di-t-butyl dicarbonate (38 g, 175 mmol). After stirring overnight, the solvent was removed in vacuo, the residue was dissolved in ethyl acetate (500 mL), and the solution was washed with satd aq sodium bicarbonate, water and then brine. The organic phase was then dried with $MgSO_4$, filtered and concentrated in vacuo to a volume of about 100 mL. The mixture was then sonicated and the precipitate was filtered and dried in vacuo to give 9.52 g (31%) of the title compound. To the mother liquor was added about 50 g of silica gel and the mixture was concentrated in vacuo. The resulting dry pack was loaded onto a silica gel column prepared with a solution of 50% ethyl acetate in hexanes and eluted with 20% ethyl acetate in dichloromethane, followed by a step gradient of 50% ethyl acetate in hexanes through ethyl acetate. The product containing fractions were combined and concentrated in vacuo to give another 16.16 g (52%) of the title compound.
$^1$H-NMR
IS-MS, m/e 195.3 (m+1)

B. 4-(Boc-amino)pyridine-3-carboxylic Acid.

To a stirring solution of 4-(Boc-amino)pyridine (1.027 g, 5.30 mmol) in THF at -36 °C (internal temperature) was added a 1.7 M solution of t-butyl lithium in pentane(6.5 mL, 11 mmol), and the rate of addition was controlled so as to keep the internal temperature below -28 °C. After an additional hour (temperature kept between -30 °C and -50 °C) carbon dioxide (g) was bubbled through the solution and the cold bath was removed. After about 15 min, the mixture was poured into ice water and the aqueous phase was washed with dichloromethane. The pH was adjusted to 4-5 with citric acid and the resulting precipitate was washed with dichloromethane and methanol and dried in vacuo to give 0.811 g (64%) of an off-white solid.
$^1$H-NMR
IS-MS, m/e 239.0 (m+1)

| Analysis for $C_{11}H_{14}N_2O_4$: | | | |
|---|---|---|---|
| Calcd | C, 55.46; | H, 5.92; | N, 11.76; |
| Found | C, 55.73; | H, 6.07; | N, 11.75. |

C. Methyl 4-(Boc-amino)pyridine-3-carboxylate.

To a stirring suspension of 4-(Boc-amino)pyridine-3-carboxylic acid (1.04 g, 4.37 mmol) in methanol (3.5 mL) was added a 2 M solution of (trimethylsilyl)diazomethane in hexanes (3.5 mL, 7 mmol). After 15 min, acetic acid was added and the solvents were removed in vacuo. The residue was chromatographed over silica gel, eluting with a step gradient of 20 % ethyl acetate in hexanes through 70 % ethyl acetate in hexanes. The product containing fractions were combined and concentrated in vacuo to give 0.894 g (81%) of a white solid.
$^1$H-NMR

D. Methyl 4-Aminopyridine-3-carboxylate.

Methyl 4-(Boc-amino)pyridine-3-carboxylate (2.38 g, 9.4 mmol) was dissolved in TFA (20 mL) and the solution was allowed to stir for 45 min. The solvent was removed in vacuo and the residue was partitioned between 25% isopropanol in chloroform and satd aq sodium bicarbonate. The layers were separated and the aqueous phase was extracted again with 25% isopropanol in chloroform. The combined organic extracts were dried ($MgSO_4$), filtered and concentrated in vacuo to give a solid which was washed with diisopropyl ether and dried in vacuo to give 1.327 g (92%) of an off-white solid.
$^1$H-NMR
IS-MS, m/e 153.1 (m+1)

| Analysis for $C_7H_8N_2O_2$: | | | |
|---|---|---|---|
| Calcd | C, 55.26; | H, 5.30; | N, 18.41; |
| Found | C, 55.31; | H, 5.36; | N, 18.42. |

E. Methyl 4-(4-t-Butylbenzoyl)aminopyridine-3-carboxylate.

To a stirring suspension of methyl 4-aminopyridine-3-carboxylate (0.161 g, 1.059 mmol), 4-dimethylaminopyridine (0.017 g, 0.138 mmol) and N,N-diisopropylethylamine (0.3 mL, 1.7 mmol) in dichloromethane (5 mL) was added 4-t-butylbenzoyl chloride (0.3 mL, 1.5 mmol). After 2 h, the mixture was diluted with ethyl acetate and satd aq sodium bicarbonate. The layers were separated and the organic phase was washed with satd aq sodium bicarbonate, then dried with $MgSO_4$, filtered and concentrated in vacuo. The residue was suspended in diisopropyl ether with vigorous stirring and the solid was filtered and dried in vacuo to give 0.257 g (78%) of a white solid.
$^1$H-NMR
IS-MS, m/e 313.0 (m+1)

| Analysis for $C_{18}H_{20}N_2O_3$: | | | |
|---|---|---|---|
| Calcd | C, 69.21; | H, 6.45; | N, 8.97; |
| Found | C, 69.43; | H, 6.37; | N, 9.15. |

F. 4-(4-t-Butylbenzoyl)aminopyridine-3-carboxylic Acid.

To a stirring solution of methyl 4-(4-t-butylbenzoyl)aminopyridine-3-carboxylate (0.156 g, 0.5 mmol) in THF (4 mL) and methanol (1 mL), was added a 1 M solution of aq LiOH (0.6 mL, 0.6 mmol). After 1 h, the solvent was removed in vacuo and the residue was partitioned between water and diethyl ether. The aqueous phase was separated and the pH was adjusted to 2-3 with citric acid. The precipitate was isolated by filtration and washed with water, with 25% isopropanol in chloroform and with diethyl ether and dried in vacuo to give 0.138 g (92%) of a white solid.
$^1$H-NMR
IS-MS, m/e 299.1 (m+1)

G. 2-(4-t-Butylphenyl)-4H-6-aza-3,1-benzoxazin-4-one.

To a stirring suspension of 4-(4-t-butylbenzoyl)aminopyridine-3-carboxylic acid (0.419 g, 1.4 mmol) in DMF (14 mL) was added 1-(3-dimethylaminopropyl)-3-ethylcarbodiimide hydrochloride (0.374 g, 1.96 mmol). After stirring overnight, the solvent was removed in vacuo and the residue was chromatographed over silica gel, eluting with a step gradient of 20% ethyl acetate in hexanes through 60% ethyl acetate in hexanes. The product containing fractions were combined and concentrated in vacuo to give 0.326 g (66%) of a white solid.
$^1$H-NMR
FD-MS, m/e 280 (m+1)

| Analysis for $C_{17}H_{16}N_2O_2$: | | | |
|---|---|---|---|
| Calcd | C, 72.84; | H, 5.75; | N, 9.99; |
| Found | C, 72.54; | H, 5.78; | N, 10.11. |

H. 4-[(4-t-Butylbenzoyl)amino]-N-(5-chloropyridin-2-yl)pyridine-3-carboxamide.

To a stirring solution of 2-amino-5-chloropyridine (0.11 g, 0.85 mmol) in THF (12 mL) at 0 °C was added a 1 M solution of allylmagnesium bromide in diethyl ether (0.83 mL, 0.83 mmol). After 20 min, 2-[4-t-butylphenyl]-4H-6-aza-3,1-benzoxazin-4-one (0.115 g, 0.41 mmol) was added and the cold bath was removed. After 2 h, the mixture was diluted with ethyl acetate and washed three times with brine. The organic phase was dried ($MgSO_4$), filtered and concentrated in vacuo. The solid was recrystallized from diethyl ether then washed several times with mixtures of ether/hexanes and dried to give 0.138 g (82%) of pale yellow needles.
$^1$H-NMR
IS-MS, m/e 409.5 (m+1)

| Analysis for $C_{22}H_{21}N_4O_2Cl$: | | | |
|---|---|---|---|
| Calcd | C, 64.62; | H, 5.18; | N, 13.70; |
| Found | C, 64.65; | H, 5.18; | N, 13.95. |

**Example 22**

**Preparation of N-(5-Chloropyridin-2-yl)-4-[(1-isopropylpiperidin-4-ylcarbonyl)amino]pyridine-3-carboxamide Dihydrochloride.**

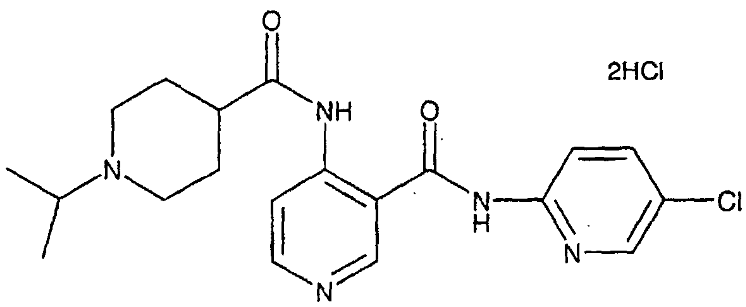

A. Methyl 4-[(1-Boc-piperidin-4-ylcarbonyl)amino]pyridine-3-carboxylate.

By methods substantially equivalent to those described in example 6-A, methyl 4-[(1-Boc-piperidin-4-ylcarbonyl)amino]pyridine-3-carboxylate (0.324 g, 84%) was prepared from methyl 1-Boc-piperidin-4-ylcarbonyl chloride and methyl 4-aminopyridine-3-carboxylate.
$^1$H-NMR
IS-MS, m/e 364.1 (m+1)

| Analysis for $C_{18}H_{25}N_3O_5$: | | | |
|---|---|---|---|
| Calcd | C, 59.49; | H, 6.93; | N, 11.56; |
| Found: | C, 59.95; | H, 7.01; | N, 11.49. |

B. 4-[(1-Boc-piperidin-4-ylcarbonyl)amino]pyridine-3carboxylic Acid.

By methods substantially equivalent to those described in example 21-F, 4-[(1-Boc-piperidin-4-ylcarbonyl)amino]pyridine-3-carboxylic acid (0.148 g, 69%) was prepared from methyl 4-[(1-Boc-piperidin-4-ylcarbonyl)amino]pyridine-3-carboxylate.
$^1$H-NMR
IS-MS, m/e 350.4 (m+1)

C. N-(5-Chloropyridin-2-yl)-4-[(1-Boc-piperidin-4-yl-carbonyl)amino]pyridine-3-carboxamide.

By methods substantially equivalent to those described in example 21-G and 21-H, N-(5-chloropyridin-2-yl)-4-[(1-Boc-piperidin-4-ylcarbonyl)amino]pyridine-3-carboxamide (0.360 g, 50% for 2 steps) was prepared from 4-[(1-Boc-piperidin-4-ylcarbonyl)amino]pyridine-3-carboxylic acid and 5-chloro-2-aminopyridine.
$^1$H-NMR
IS-MS, m/e 460.4 (m+1)

| Analysis for $C_{22}H_{26}ClN_5O_4$: | | | | |
|---|---|---|---|---|
| Calcd | C, 57.45; | H, 5.70; | N, 15.23; | Cl, 7.71; |
| Found | C, 57.15; | H, 5.78; | N, 14.88; | Cl, 8.09. |

D. N-(5-Chloropyridin-2-yl)-4-[(piperidin-4-ylcarbonyl)amino]pyridine-3-carboxamide trifluoroacetate.

By methods substantially equivalent to those described in example 6-B, N-(5-chloropyridin-2-yl)-4-[(piperidin-4-yl-carbonyl)amino]pyridine-3-carboxamide trifluoroacetate (53 mg, 71%) was prepared from N-(5-chloropyridin-2-yl)-4-[(1-Boc-piperidin-4-ylcarbonyl)amino]pyridine-3-carboxamide.
$^1$H-NMR
IS-MS, m/e 360.1 (m+1)

E. N-(5-Chloropyridin-2-yl)-4-[(1-isopropylpiperidin-4-yl-carbonyl)amino]pyridine-3-carboxamide Hydrochloride.

By methods substantially equivalent to those described in example 6-C, N-(5-chloropyridin-2-yl)-4-[(1-isopropylpiperidin-4-ylcarbonyl)amino]pyridine-3-carboxamide hydrochloride (0.219 g, 50%) was prepared from N-(5-chloropyridin-2-yl)-4-[(piperidin-4-ylcarbonyl)amino]pyridine-3-carboxamide trifluoroacetate and acetone. The compound was purified by preparative RPHPLC (C18), eluting with a linear gradient of 95/5 to 60/40 (0.01% HCl/acetonitrile) over 200 min.
$^1$H-NMR
FD-MS, m/e 402.3 (m+1)

| Analysis for $C_{20}H_{24}N_5O_2Cl \cdot 2.4HCl \cdot 3.6H_2O$: | | | | |
|---|---|---|---|---|
| Calcd | C, 43.34; | H, 6.11; | N, 12.64; | Cl, 21.75; |
| Found | C, 43.55; | H, 5.90; | N, 12.32; | Cl, 21.91. |

**Example 23**

**Preparation of N-(5-Chloropyridin-2-yl)-2-[(1-isopropylpiperidin-4-ylmethyl)amino]pyridine-3-carboxamide Trihydrochloride.**

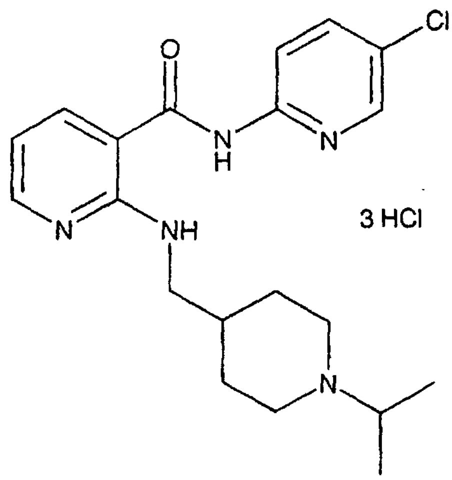

A. 2-Chloro-N-(5-chloropyridin-2-yl)nicotinamide.

By methods substantially equivalent to those described in example 14-A, 2-chloro-N-(5-chloropyridin-2-yl) nicotinamide was prepared from 2-amino-5-chloropyridine and 2-chloronicotinoyl chloride.

B. N-(5-Chloropyridin-2-yl)-2-[(1-isopropylpiperidin-4-ylmethyl)amino]pyridine-3-carboxamide Hydrochloride.

A solution of 0.57 g of 2-chloro-N-(5-chloropyridin-2-yl)nicotinamide, in 7 mL of pyridine, was treated with 0.64 g of 1-isopropylpiperidine-4-methylamine, and the mixture was refluxed 68 h. The mixture was cooled to room temperature and then treated with 1 mL of 5 N NaOH and evaporated to dryness. The EtOH extract was purified by radial chromatography (10% MeOH-$CHCl_3$, 1% $NH_4OH$) to give 0.25 g of free base. The HCl salt was isolated as an amorphous foam (0.21 g, 19%).
$^1$H-NMR
IS-MS, m/e 388 (m+1)

| Analysis for $C_{20}H_{26}ClN_5O \cdot 3HCl \cdot 1.75H_2O$: | | | |
|---|---|---|---|
| Calcd | C, 45.42; | H, 6.19; | N, 13.24; |
| Found | C, 45.64; | H, 5.97; | N, 12.85. |

**Example 24**

**Preparation of N-(5-Chloropyridin-2-yl)-3-[(1-isopropylpiperidin-4-ylcarbonyl)amino]-6-methylpyridine-2-carboxamide Hydrochloride.**

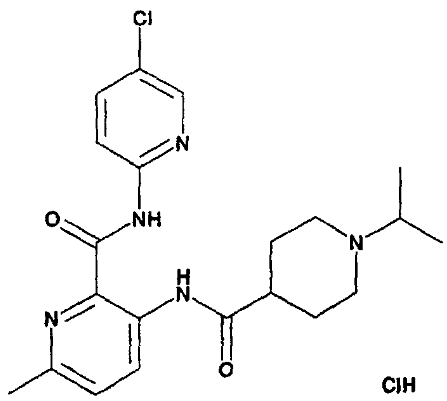

A. 3-Amino-N-(5-chloropyridin-2-yl)-6-methylpyridine-2-carboxamide.

Using methods substantially equivalent to those described in Example 2-A, 3-amino-N-(5-chloropyridin-2-yl)-6-methylpyridine-2-carboxamide (16 g, 46%) was prepared from 3-amino-2-chloro-6-methylpyridine and 2-amino-5-chloropyridine.
$^1$H NMR
FIA-MS, m/e 263.1 (m+1).

B. 3-[(1-tert-Butoxycarbonylpiperidin-4-ylcarbonyl)amino]-N-(5-chloropyridin-2-yl)-6-methylpyridine-2-carboxamide.

Using methods substantially equivalent to those described in Example 6-A, 3-[(1-tert-butoxycarbonylpiperidin-4-ylcarbonyl)amino]-N-(5-chloropyridin-2-yl)-6-methylpyridine-2-carboxamide (1.26 g, 93%) was prepared from 3-amino-N-(5-chloropyridin-2-yl)-6-methylpyridine-2-carboxamide (0.75 g, 2.85 mmol) and N-Boc-isonipecotic acid. The crude product was purified by chromatography over silica gel, eluting with a step gradient of 5-15% ethyl acetate in dichloromethane.
$^1$H NMR
IS-MS, m/e 474.1 (m+1), 472.3 (m-1)

| Analysis for $C_{23}H_{28}ClN_5O_4$: | | | |
|---|---|---|---|
| Calcd | C, 58.29; | H, 5.95; | N, 14.78; |
| Found | C, 58.01; | H, 5.90; | N, 14.90. |

C. N-(5-Chloropyridin-2-yl)-6-methyl-3-[(4-piperidinylcarbonyl)amino]pyridine-2-carboxamide trifluoroacetate.

To a stirring solution of 3-[(1-tert-butoxycarbonylpiperidin-4-ylcarbonyl)amino]-N-(5-chloropyridin-2-yl)-6-methylpyridine-2-carboxamide (0.88 g, 1.86 mmol) and anisole (1.0 mL) in dichloromethane (40 mL) was added TFA (3.6 mL). After stirring for 4 h, the solvent was removed in vacuo and the residue was suspended in diethyl ether with vigorous stirring. After 30 min, the solid was filtered, washed several times with diethyl ether and then dried in vacuo to give (0.90 g, 99%) of a white solid.
$^1$H NMR
IS-MS, m/e 374.1 (m+1), 372.1 (m-1)

| Analysis for $C_{18}H_{20}ClN_5O_2 \cdot 1.2C_2HF_3O_2 \cdot 1.2H_2O$: | | | | |
|---|---|---|---|---|
| Calcd | C, 46.03; | H, 4.47; | N, 13.16; | F, 12.85; |

(continued)

| Analysis for $C_{18}H_{20}ClN_5O_2 \cdot 1.2C_2HF_3O_2 \cdot 1.2H_2O$: | | | | |
|---|---|---|---|---|
| Found | C, 46.21; | H, 4.08; | N, 12.97; | F, 12.36. |

D. N-(5-Chloropyridin-2-yl)-3-[(1-isopropylpiperidin-4-ylcarbonyl)amino]-6-methylpyridine-2-carboxamide hydrochloride.

To a stirring suspension of N-(5-chloropyridin-2-yl)-6-methyl-3-[(4-piperidinylcarbonyl)amino]pyridine-2-carboxamide trifluoroacetate (0.75 g, 1.54 mmol) in methanol (11 mL) was added acetone (11 mL), followed by acetic acid (0.45 mL, 7.86 mmol), and then sodium cyanoborohydride (0.51 g, 7.7 mmol). After stirring overnight, the solution was treated with saturated aqueous ammonium chloride solution, concentrated, and partitioned between dichloromethane and saturated aqueous sodium bicarbonate. The organic phase was washed with brine, dried over magnesium sulfate, filtered, and concentrated. The crude product was purified by chromatography over silica gel, eluting with a step gradient of 0-10% 2 M solution of ammonia/methanol in dichloromethane. To a stirring solution of the purified product in dichloromethane was added 1.0 N hydrochloric acid in diethyl ether until precipitate formed. The mixture was filtered to give 0.28 g (40%) of a white solid.
$^1$H NMR
IS-MS, m/e 416.2 (m+1), 414.2 (m-1)

| Analysis for $C_{21}H_{26}ClN_5O_2 \cdot 1.8HCl \cdot 0.4H_2O$: | | | | |
|---|---|---|---|---|
| Calcd | C, 51.60; | H, 5.90; | N, 14.33; | Cl, 20.31; |
| Found | C, 51.88; | H, 5,73; | N, 14.25; | Cl, 20.54. |

**Example 25**

**Preparation of N-(5-Chloropyridin-2-yl)-3-[(1-isopropylpiperidin-4-ylmethyl)amino]pyrazine-2-carboxamide.**

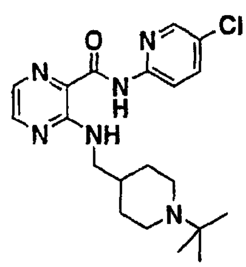

A. 2-Chloro-3-[(1-isopropylpiperidin-4-ylmethyl)amino]pyrazine.

A mixture of of 2,3-dichloropyrazine (5.0 g, 32 mmol), 4-aminomethyl-1-isopropylpiperidine (5.3 g, 34 mmol), and pyridine (3 mL) in dry toluene (50 mL) was heated at 60 °C for 19 h. The cooled mixture was filtered and the filtrate was evaporated. This residue was refiltered from ether to give 6.0 g of crude product. Flash column purification with 10% MeOH-$CHCl_3$, 1% $NH_4OH$ gave 2.9 g (33%)of the desired product.

B. N-(5-Chloropyridin-2-yl)-3-[(1-isopropylpiperidin-4-ylmethyl)amino]pyrazine-2-carboxamide.

A carbonylation mixture of 2-chloro-3-[(1-isopropylpiperidin-4-ylmethyl)amino]pyrazine (1.37 g, 5.10 mmol), 2-amino-5-chloropyridine (4.1 g, 3.19 mmol), 1,3-bis(diphenylphosphino)propane (1.3 g, 3.15 mmol), palladium acetate (0.24 g, 1.1 mmol),and triethylamine (2.2 g, 21.7 mmol), in acetonitrile under a 54.4 bar carbon monoxide atmosphere was heated at 100 °C for 72 h. The reaction mixture was filtered and evaporated to give 6.74 g crude product. After basification with NaOH, extraction with ethyl acetate and evaporation of the organic phase, the residue was purified

by flash-column chromatography over silica gel, eluting with 2.5% MeOH-$CHCl_3$, 0.25% $NH_4OH$, to give 1.43 g (72%) of the desired product.

$^1$NMR (300 MHz, $CDCl_3$) δ 10.40 (s, 1H); 8.59 (s, 1H); 8.28 (d, J = 8.4 Hz, 1H); 8.30 (d, J = 0.7 Hz, 1H); 8.23 (d, J = 2.2 Hz, 1H); 7.74 (d, J = 2.2 Hz, 1H); 7.70 (dd, J = 6.2 Hz, 1H); 3.45 (dd, J = 6.2 Hz, 2H); 2.96 (d, J = 2.7 Hz, 2H); 2.78 (m, 1H); 2.20 (m, 2H); 1.88 (s, 1H); 1.84 (s, 1H); 1.66 (m, 1H); 1.45 (m, 2H); 1.08 (d. J = 6.2 Hz, 6H).

IS-MS, m/e = 389.3 (m+1)

| Analysis for $C_{19}H_{25}ClN_6O{\cdot}0.25\ H_2O$: | | | |
|---|---|---|---|
| Calcd | C, 58.00; | H, 6.53; | N, 21.36; |
| Found | C, 58.19; | H, 6.36; | N, 20.84. |

## Claims

**1.** A compound of formula I

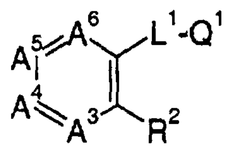

I

(or a pharmaceutically acceptable salt thereof) wherein:

$A^3$, $A^4$, $A^5$ and $A^6$, together with the two carbons to which they are attached, complete a substituted heteroaromatic ring in which

(a) one of $A^3$, $A^4$, $A^5$ and $A^6$ is N, and each of the others is $CR^3$, $CR^4$, $CR^5$ or $CR^6$, respectively; or
(b) two non-adjacent residues of $A^3$, $A^4$, $A^5$ and $A^6$ are each N, and each of the others is $CR^3$, $CR^4$, $CR^5$ or $CR^6$, respectively; wherein

each of $R^3$, $R^4$, $R^5$ and $R^6$ is independently hydrogen or methyl, or one of $R^3$, $R^4$, $R^5$ and $R^6$ attached to a carbon which is not bonded to an N-atom is chloro and the others are hydrogen;
$L^1$ is -CO-NH- such that $-L^1-Q^1$ is $-CO-NH-Q^1$;
$Q^1$ is 2-pyridinyl (which bears a methyl, methoxy, methylthio, fluoro or chloro substituent at the 5-position), 3-pyridinyl (which bears a methyl, fluoro or chloro substituent at the 6-position), 2-pyrimidinyl (which may bear a methyl, fluoro or chloro substituent at the 5-position), 3-pyridazinyl (which may bear a methyl, fluoro or chloro substituent at the 6-position) or 2-benzothiazolyl (which may bear a methyl, fluoro, chloro or bromo substituent at the 6-position);
$R^2$ is $-L^2-Q^2$ in which $-L^2-$ is -NH-CO-, -NH-CO-X-, -NH-CO-O-X-, -NH-CO-NH-X- or $-NH-CH_2-$; and $Q^2$ is $Q^{2A}$, $Q^{2B}$, $Q^{2C}$, $Q^{2D}$, $Q^{2E}$ or $Q^{2F}$ wherein X is a single bond or methylene and the values of $L^2$ and $Q^2$ are together selected from $-NH-CO-X-Q^{2A}$, $-NH-CO-O-X-Q^{2A}$, $-NH-CO-NH-X-Q^{2A}$ $-NH-CH_2-Q^{2A}$, $-NH-CO-X-Q^{2B}$, $-NH-CO-Q^{2C}$, $-NH-CO-Q^{2D}$, $-NH-CO-Q^{2E}$ and $-NH-CO-Q^{2F}$ in which:

$Q^{2A}$ (showing the $L^2$ to which it is attached) is

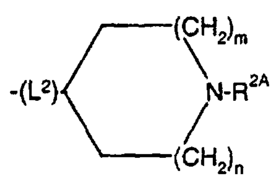

in which
each of m and n independently is 0 or 1, and
$R^{2A}$ is hydrogen, t-butyl, methylsulfonyl, $-CHR^yR^z$, $-CHR^wR^x$, or 4-pyridinyl (which is unsubstituted or bears a substituent $R^v$ at the 2- or 3-position) wherein
$R^v$ is methyl, hydroxymethyl, {(1-2C)alkoxy}carbonyl; cyano, carbamoyl, thiocarbamoyl, or N-hydroxyamidino;
each of $R^w$ and $R^x$ independently is hydrogen or (1-3C)normal alkyl; or $-CHR^wR^x$ is 2-indanyl or (showing the nitrogen to which it is attached) is

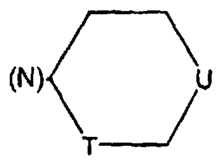

in which T is a single bond or methylene and U is methylene, ethylene, oxy, $-S(O)_q-$ (wherein q is 0, 1 or 2) or imino (which may bear a methyl substituent), or T is ethan-1,1-diyl and U is a single bond or methylene;
$R^y$ is hydrogen or methyl; and
$R^z$ is isopropyl, t-butyl, (3-6C)cycloalkyl, phenyl (which is unsubstituted or bears one or more substituents independently selected from halo, methyl, methoxy and hydroxy), 4-quinolinyl or heteroaryl (which heteroaryl is a 5-membered aromatic ring which includes one to four heteroatoms selected from sulfur, oxygen and nitrogen or is a 6-membered aromatic ring which includes one to three nitrogen atoms, wherein the heteroaryl is attached at carbon and may bear one or more methyl substituents on carbon or nitrogen);
$Q^{2B}$ is 1-piperazinyl which bears at the 4-position the group $R^{2A}$ (defined as above);
$Q^{2C}$ is 3,4-didehydropiperidin-4-yl which bears at the 1-position the group $R^{2A}$ (defined as above);
$Q^{2D}$ is cyclohexyl which bears at the 4-position the group $-NR^sR^t$ in which each of $R^s$ and $R^t$ independently is hydrogen or methyl or $R^s$ and $R^t$ together are trimethylene or tetramethylene;
$Q^{2E}$ is 1-piperidinyl which bears at the 4-position the group $-NR^sR^t$ (defined as above) ; and
$Q^{2F}$ (showing the $L^2$ to which it is attached) is

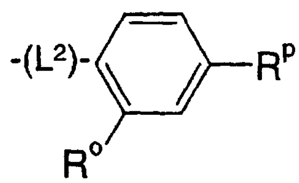

in which $R^o$ is hydrogen, halo, (1-6C)alkyl, hydroxy, (1-4C)alkoxy, benzyloxy or (1-4C)alkylthio; and $R^p$ is 1-hydroxyethyl, 1-hydroxy-1-methylethyl, 1-methoxy-1-methylethyl, 4-piperidinyl, 4-pyridinyl, dimethylaminosulfonyl or $-J-R^q$ in which J is a single bond, methylene, carbonyl, oxy, $-S(O)_q-$ (wherein q is 0, 1 or 2), or $-NR^r-$ (wherein $R^r$ is hydrogen or methyl); and $R^q$ is (1-6C)alkyl, phenyl, 3-pyridyl or 4-pyridyl.

**2.** The compound of Claim 1 wherein halo is fluoro, chloro, bromo or iodo; (1-2C)alkyl is methyl or ethyl; (1-3C) normal alkyl is methyl, ethyl or propyl; (1-4C)alkyl is methyl, ethyl, propyl, isopropyl, butyl, isobutyl, or t-butyl; (1-6C)alkyl is methyl, ethyl, propyl, butyl, pentyl or hexyl; (3-6C)cycloalkyl is cyclopropyl, cyclobutyl, cyclopenytyl or cyclohexyl.

**3.** The compound of Claim 1 or 2 wherein $Q^1$ is 5-chloropyridin-2-yl or 6-chloropyridazin-3-yl.

**4.** The compound of any of Claims 1-3 wherein $R^2$ is (1-isopropylpiperidin-4-ylcarbonyl)amino, (1-cyclohexylpiperidin-4-ylcarbonyl)amino, [1-(tetrahydropyran-4-yl)piperidin-4-ylcarbonyl]amino, or [1-(4-pyridinyl)piperidin-4-ylmethyl] amino.

**5.** The compound as claimed in any of Claims 1-4 wherein $A^3$ is N and each of $A^4$-$A^6$ is $CR^4$-$CR^6$ in which each of $R^4$-$R^6$ is hydrogen or $R^4$ and $R^6$ are each hydrogen and $R^5$ is chloro.

**6.** The compound as claimed in any of Claims 1-4 wherein $A^6$ is N and each of $A^3$-$A^5$ is $CR^3$-$CR^5$ in which each of $R^3$-$R^5$ is hydrogen or $R^3$ and $R^4$ are each hydrogen and $R^5$ is methyl.

**7.** The pharmaceutically acceptable salt of a compound of formula I as claimed in any of Claims 1-6 which is an acid-addition salt made from a basic compound of formula I and an acid which provides a pharmaceutically acceptable anion or a salt which is made from an acidic compound of formula I and a base which provides a pharmaceutically acceptable cation.

**8.** A pharmaceutical formulation comprising in association with a pharmaceutically acceptable carrier, diluent or excipient, a novel compound of formula I (or a pharmaceutically acceptable salt thereof) as provided in any of Claims 1-7.

**9.** A process for preparing a compound of formula I (or a pharmaceutically acceptable salt thereof) as provided in any of the above descriptions which is selected from

(A) for a compound of formula I in which $-L^2-Q^2$, is $-NH-CO-Q^2$, $-NH-CO-X-Q^2$, $-NH-CO-O-X-Q^2$ or $-NH-CO-NH-X-Q^2$, acylating an amine of formula II,

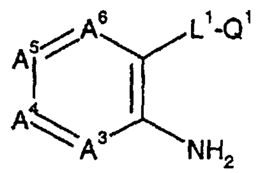

II

using a corresponding acid of formula $HO-CO-Q^2$, $HO-CO-X-Q^2$, $HO-CO-O-X-Q^2$, or $HO-CO-NH-X-Q^2$, or an activated derivative thereof;

(B) for a compound of formula I in which $-L^2-Q^2$ is $-NH-CH_2-Q^2$, substituting the group $Y^a$ of a compound of formula III

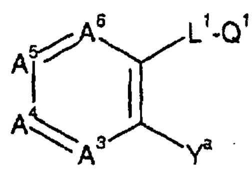

III

in which $Y^a$ is a conventional leaving group for nucleophilic aromatic substitution with an amine of formula $NH_2-CH_2-Q^2$;

(C) acylating an amine of formula $H_2N-Q^1$, or a deprotonated derivative thereof, using an acid of formula IV, or an activated derivative thereof;

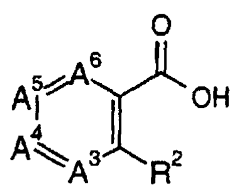

IV

(D) for a compound of formula I in which $R^2$ is $-NH-CH_2-Q^{2A}$, alkylating an amine of formula II directly, using a compound of formula $Y-CH_2-Q^{2A}$, or indirectly by reductive alkylation using an aldehyde of formula $Q^{2A}-CHO$;

(E) for a compound of formula I in which $R^2$ is $-NH-CO-O-X-Q^{2A}$, or $-NH-CO-NH-X-Q^{2A}$, acylating an alcohol of formula $HO-X-Q^{2A}$ or an amine of formula $NH_2-X-Q^{2A}$, using an activated derivative of an acid of formula VI;

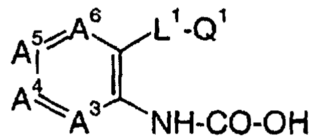

VI

(F) for a compound of formula I in which $R^2$ is -NH-CO-X-$Q^{2B}$ in which X is a single bond, acylating at the 1-position a piperazine of formula H-$Q^{2B}$, using an activated derivative of an acid of formula VI;

(G) for a compound of formula I in which $R^2$ is -NH-CO-X-$Q^{2B}$ in which X is methylene, alkylating at the 1-position a piperazine of formula H-$Q^{2B}$, using an alkylating agent of formula VII

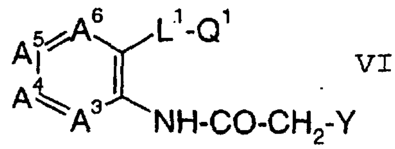

VII

in which Y is a leaving group;

(H) for a compound of formula I in which $R^{2A}$ is methylsulfonyl, substituting the amino nitrogen of a corresponding compound of formula I in which $R^{2A}$ is hydrogen using an activated derivative of methanesulfonic acid;

(I) for a compound of formula I in which $R^{2A}$ is -$CHR^yR^z$ or -$CHR^wR^x$, alkylating the amino nitrogen of a corresponding compound of formula I in which $R^{2A}$ is hydrogen using an alkylating agent of formula Y-$CHR^yR^z$ or Y-$CHR^wR^x$ or reductively alkylating the amine using a compound of formula $R^y$-CO-$R^z$ or $R^w$CO-$R^x$-;

(J) for a compound of formula I in which $R^{2A}$ is 4-pyridinyl (which is unsubstituted or bears a substituent $R^v$ at the 2- or 3-position), substituting the amino nitrogen of a corresponding compound of formula I in which $R^{2A}$ is hydrogen using a corresponding pyridine reagent bearing a leaving group Y at the 4-position;

(K) for a compound of formula I in which $R^{2A}$ is 4-pyridinyl in which $R^v$ is alkoxycarbonyl, esterifying a corresponding compound of formula I in which $R^v$ is carboxy;

(L) for a compound of formula I in which $R^{2A}$ is 4-pyridinyl in which $R^v$ is hydroxymethyl, reducing the ester of a corresponding compound of formula I in which $R^v$ is alkoxycarbonyl;

(M) for a compound of formula I in which $R^{2A}$ is 4-pyridinyl in which $R^v$ is carbamoyl, amidating the ester of a corresponding compound of formula I in which $R^v$ is alkoxycarbonyl;

(N) for a compound of formula I in which $R^{2A}$ is 4-pyridinyl in which $R^v$ is thiocarbamoyl, adding $H_2S$ to the nitrile of a corresponding compound of formula I in which $R^v$ is cyano;

(O) for a compound of formula I in which $R^{2A}$ is 4-pyridinyl in which $R^v$ is N-hydroxyamidino, adding $H_2NOH$ to the nitrile of a corresponding compound of formula I in which $R^v$ is cyano;

(P) for a compound of formula I in which $R^{2A}$ is 4-pyridinyl in which $R^v$ is carboxy, decomposing the ester of a corresponding compound of formula I in which $R^v$ is alkoxycarbonyl;

(Q) for a compound of formula I in which -$NR^sR^t$ is other than amino, alkylating a corresponding compound of formula I in which -$NR^sR^t$ is amino using a conventional method;

(R) for a compound of formula I which bears -$NR^sR^t$, reductively alkylating H-$NR^sR^t$ using a corresponding compound but in which the carbon to bear the -$NR^sR^t$ group bears an oxo group;

(S) for a compound of formula I in which RP is 1-hydroxy-1-methylethyl, adding a methyl group to the carbonyl group of a corresponding compound of formula I in which $R^p$ is acetyl using an organometallic reagent;

(T) for a compound of formula I in which $R^p$ is 1-methoxy-1-methylethyl, treating a corresponding compound of formula I in which $R^p$ is 1-hydroxy-1-methylethyl with methanol and an acid catalyst;

whereafter, for any of the above procedures, when a functional group is protected using a protecting group, removing the protecting group;

whereafter, for any of the above procedures, when a pharmaceutically acceptable salt of a compound of formula I is required, it is obtained by reacting the basic form of a basic compound of formula I with an acid affording a physiologically acceptable counterion or the acidic form of an acidic compound of formula I with a base affording a physiologically acceptable counterion or by any other conventional procedure;

and wherein, unless otherwise specified, $A^3$-$A^6$, $L^1$, $Q^1$ and $R^2$ have any of the values defined in Claim 1.

**10.** Use of a compound of formula I according to any one of Claims 1 to 7 in the manufacture of a medicament for inhibiting factor Xa in a mammal.

## Patentansprüche

**1.** Verbindung der Formel I

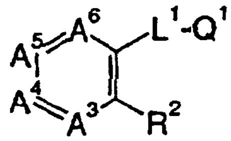

I

(oder ein pharmazeutisch annehmbares Salz hiervon), worin

$A^3$, $A^4$, $A^5$ und $A^6$ zusammen mit den zwei Kohlenstoffen, an die sie gebunden sind, einen substituierten, heteroaromatischen Ring bilden, worin

(a) eines von $A^3$, $A^4$, $A^5$ und $A^6$ für N steht und die anderen jeweils für $CR^3$, $CR^4$, $CR^5$ oder $CR^6$ stehen, oder
(b) zwei nicht-benachbarte Reste von $A^3$, $A^4$, $A^5$ und $A^6$ jeweils für N stehen und jedes der anderen jeweils für $CR^3$, $CR^4$, $CR^5$ oder $CR^6$ steht, worin

jedes von $R^3$, $R^4$, $R^5$ und $R^6$ unabhängig für Wasserstoff oder Methyl steht, oder eines von $R^3$, $R^4$, $R^5$ und $R^6$ an einen Kohlenstoff gebunden ist, der nicht an ein N-Atom gebunden ist, für Chlor steht und die anderen für Wasserstoff stehen,

$L^1$ für -CO-NH- steht, so daß $-L^1-Q^1$ für $-CO-NH-Q^1$ steht,

$Q^1$ für 2-Pyridinyl (das einen Methyl-, Methoxy-, Methylthio-, Fluor- oder Chlorsubstituenten an der Position 5 trägt), 3-Pyridinyl (das einen Methyl-, Fluor- oder Chlorsubstituenten an der Position 6 trägt), 2-Pyrimidinyl (das einen Methyl-, Fluro- oder Chlorsubstituenten an der Position 5 trägt), 3-Pyridazinyl (das einen Methyl-, Fluor- oder Chlorsubstituenten an der Position 6 trägt) oder 2-Benzothiazolyl (das einen Methyl-, Fluor-, Chlor- oder Bromsubstituenten an der Position 6 trägt) steht,

$R^2$ für $-L^2-Q^2$ steht, worin $-L^2-$ für -NH-CO-, -NH-CO-X-, -NH-CO-O-X-, -NH-CO-NH-X- oder $-NH-CH_2-$ steht und $Q^2$ für $Q^{2A}$, $Q^{2B}$, $Q^{2C}$, $Q^{2D}$, $Q^{2E}$ oder $Q^{2F}$ steht, worin X für eine Einfachbindung oder Methylen steht und die Bedeutungen für $L^2$ und $Q^2$ zusammen ausgewählt sind aus $-NH-CO-X-Q^{2A}$, $-NH-CO-O-X-Q^{2A}$, $-NH-CO-NH-X-Q^{2A}$, $-NH-CH_2-Q^{2A}$, $-NH-CO-X-Q^{2B}$, $-NH-CO-X-Q^{2C}$, $-NH-CO-X-Q^{2D}$, $-NH-CO-X-Q^{2E}$ und $-NH-CO-X-Q^{2F}$ steht, worin

$Q^{2A}$ steht für (L2, an das es gebunden ist, wird auch gezeigt)

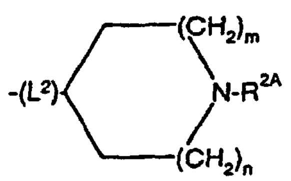

worin
jedes von m und n unabhängig für 0 oder 1 steht und

$R^{2A}$ für Wasserstoff, t-Butyl, Methansulfonyl, $-CHR^YR^Z$, $-CHR^WR^X$ oder 4-Pyridinyl steht (das unsubstituiert ist oder einen Substituenten $R^V$ an der Position 2 oder 3 trägt), worin

$R^V$ für Methyl, Hydroxymethyl, $(C_1-C_2)$ Alkoxycarbonyl, Cyano, Carbamoyl, Thiocarbamoyl oder N-Hydroxyamidino steht, jedes von $R^W$ und $R^X$ unabhängig fiir Wasserstoff oder normales

$C_1$-$C_3$ Alkyl steht oder -$CHR^WR^X$ für 2-Indanyl oder folgendes steht (wobei der Stickstoff, an den es gebunden ist, gezeigt wird)

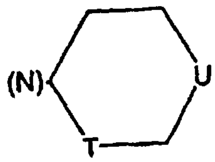

worin

T für eine Einfachbindung oder Methylen steht und U fiir Methylen, Ethylen, Oxy, -$S(O)_q$- (worin q für 0, 1 oder 2 steht) oder Imino steht (das einen Methylsubstituenten tragen kann) oder T für Ethan-1,1-diyl steht und U für eine Einfachbindung oder Methylen steht,

$R^y$ fiir Wasserstoff oder Methyl steht, und

$R^z$ steht fiir Isopropyl, t-Butyl, $C_3$-$C_6$ Cycloalkyl, Phenyl (das unsubstituiert ist oder einen oder mehrere Substituenten trägt, die unabhängig ausgewählt sind aus Halogen, Methyl, Methoxy und Hydroxy), 4-Chinolinyl oder Heteroaryl (wobei Heteroaryl ein fünfgliedriger, aromatischer Ring ist, der ein bis vier Heteroatome umfaßt, ausgewählt aus Schwefel, Sauerstoff und Stickstoff oder ein sechsgliedriger aromatischer Ring ist, der ein bis drei Stickstoffatome umfaßt, worin das Heteroaryl an den Kohlenstoff gebunden ist und einen oder mehrere Methylsubstituenten am Kohlenstoff oder am Stickstoff tragen kann),

$Q^{2B}$ für 1-piperazinyl steht, das an der Position 4 die Gruppe $R^{2A}$ trägt (wie oben definiert),

$Q^{2C}$ fiir 3,4-Didehydropiperidin-4-yl steht, das an der Position 1 die Gruppe $R^{2A}$ trägt (wie oben definiert),

$Q^{2D}$ für Cyclohexyl steht, das an der Position 4 die Gruppe -$NR^sR^t$ trägt, worin jedes von $R^s$ und $R^t$ unabhängig für Wasserstoff oder Methyl steht oder $R^s$ und $R^t$ zusammen für Trimethylen oder Tetramethylen stehen,

$Q^{2E}$ für 1-Piperidinyl steht, das an der Position 4 die Gruppe -$NR^sR^t$ trägt (wie oben definiert), und

$Q^{2F}$ für folgendes steht (wobei $L^2$ gezeigt ist, an das es gebunden ist)

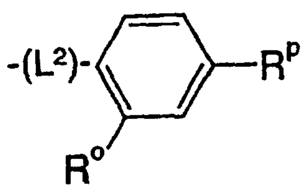

worin

$R^O$ für Wasserstoff, Halogen, $C_1$-$C_6$ Alkyl, Hydroxy, $C_1$-$C_4$ Alkoxy, Benzyloxy oder $C_1$-$C_4$ Alkylthio steht und $R^p$ für 1-Hydroxyethyl, 1-Hydroxy-1-methylethyl, 1-Methoxy-1-methylethyl, 4-Piperidinyl, 4-Pyridinyl, Dimethylaminosulfonyl oder -J-$R^q$ steht, worin J für eine Einfachbindung, Methylen, Carbonyl, Oxy, -$S(O)_q$- (worin q fiir 0, 1 oder 2 steht) oder -$NR^r$- steht (worin $R^r$ für Wasserstoff oder Methyl steht) und $R^q$ für $C_1$-$C_6$ Alkyl, Phenyl, 3-Pyridyl oder 4-pyridyl steht.

**2.** Verbindung nach Anspruch 1, worin Halogen für Fluor, Chlor, Brom oder lod steht, $C_1$-$C_2$ Alkyl für Methyl oder Ethyl steht, normales $C_1$-$C_3$ Alkyl fiir Methyl, Ethyl oder Propyl steht, $C_1$-$C_4$ Alkyl fiir Methyl, Ethyl, Propyl, Isopropyl, Butyl, Isobutyl oder t-Butyl steht, $C_1$-$C_6$ Alkyl für Methyl, Ethyl, Propyl, Butyl, Pentyl oder Hexyl steht, $C_3$-$C_6$ Cycloalkyl für Cyclopropyl, Cyclobutyl, Cyclopentyl oder Cyclohexyl steht.

**3.** Verbindung nach Anspruch 1 oder 2, worin $Q^1$ für 5-Chlorpyridin-2-yl oder 6-Chlorpyridazin-3-yl steht.

**4.** Verbindung nach einem der Ansprüche 1-3, worin $R^2$ für
(1-Isopropylpiperidin-4-ylcarbonyl)amino,
(1-Cyclohexylpiperidin-4-ylcarbonyl)amino,

[1-(Tetrahydropyran-4-yl)-piperidin-4-ylcarbonyl)amino oder [1-(4-Pyridinyl)-piperidin-4-ylmethyl)amino steht.

**5.** Verbindung nach einem der Ansprüche 1-4, worin $A^3$ für N steht und jedes von $A^4$-$A^6$ für $CR^4$-$CR^6$ steht, worin jedes von $R^4$-$R^6$ für Wasserstoff steht oder $R^4$ und $R^6$ jeweils für Wasserstoff stehen und $R^5$ für Chlor steht.

**6.** Verbindung nach einem der Ansprüche 1-4, worin $A^6$ für N steht und jedes von $A^3$-$A^5$ für $CR^3$-$CR^5$ steht, worin jedes von $R^3$-$R^5$ für Wasserstoff steht und $R^3$ und $R^4$ jeweils für Wasserstoff stehen und $R^5$ für Methyl steht.

**7.** Pharmazeutisch annehmbares Salz einer Verbindung der Formel I nach einem der Ansprüche 1-6, das ein Säureadditionssalz ist, das aus einer basischen Verbindung der Formel I und einer Säure hergestellt wurde, die ein pharmazeutisch annehmbares Anion bereitstellt, oder ein Salz, das aus einer sauren Verbindung der Formel I und einer Base hergestellt wurde, die ein pharmazeutisch annehmbares Kation bereitstellt.

**8.** Pharmazeutische Formulierung, die zusammen mit einem pharmazeutisch annehmbaren Träger, Verdünnungsmittel oder Hilfsstoff eine neue Verbindung der Formel I (oder ein pharmazeutisch annehmbares Salz hiervon) nach einem der Ansprüche 1-7 enthält.

**9.** Verfahren zur Herstellung einer Verbindung der Formel I (oder eines pharmazeutisch annehmbaren Salzes hiervon) nach einer der obigen Beschreibungen, ausgewählt aus

(A) für eine Verbindung der Formel I, worin $-L^2-Q^2$ für $-NH-CO-Q^2$, $-NH-CO-X-Q^2$, $-NH-CO-O-X-Q^2$ oder $-NH-CO-NH-X-Q^2$ steht, Acylierung eines Amins der Formel II

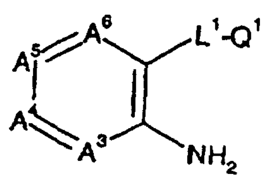

II

unter Verwendung einer entsprechenden Säure der Formel $HO-CO-Q^2$, $HO-CO-X-Q^2$, $HO-CO-O-X-Q^2$ oder $HO-CO-NH-X-Q^2$ oder eines aktivierten Derivats hiervon,

(B) für eine Verbindung der Formel I, worin $-L^2-Q^2$ für $-NH-CH_2-Q^2$ steht, Substitution der Gruppe $Y^a$ einer Verbindung der Formel III

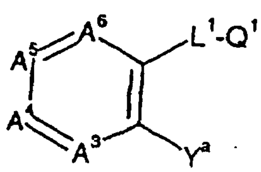

III

worin $Y^a$ für eine herkömmliche Abgangsgruppe für eine nukleophile, aromatische Substitution steht, mit einem Amin der Formel $NH_2-CH_2-Q^2$,

(C) Acylierung eines Amins der Formel $H_2N-Q^1$ oder eines deprotonierten Derivats hiervon mit einer Säure der Formel IV oder eines aktivierten Derivats hiervon

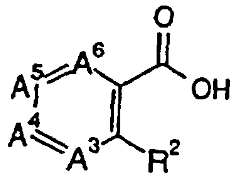

IV

(D) für eine Verbindung der Formel I, worin $R^2$ für $-NH-CH_2-Q^{2A}$ steht, Alkylierung eines Amins der Formel II direkt mittels einer Verbindung der Formel $Y-CH_2-Q^{2A}$ oder indirekt durch reduktive Alkylierung mittels eines Aldehyds der Formel $Q^{2A}$-CHO,

(E) für eine Verbindung der Formel I, worin $R^2$ für $-NH-CO-O-X-Q^{2A}$ oder $-NH-CO-NH-X-Q^{2A}$ steht, Acylierung eines Alkohols der Formel $HO-X-Q^{2A}$ oder eines Amins der Formel $NH_2-X-Q^{2A}$ mit einem aktivierten Derivat einer Säure der Formel VI

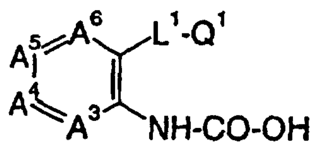

VI

(F) für eine Verbindung der Formel I, worin $R^2$ für $-NH-CO-X-Q^{2B}$ steht, worin X für eine Einfachbindung steht, Acylierung eines Piperazins der Formel $H-Q^{2B}$ an der Position 1 mittels eines aktivierten Derivats einer Säure der Formel VI,

(G) für eine Verbindung der Formel I, worin $R^2$ für $-NH-CO-X-Q^{2B}$ steht, worin X für Methylen steht, Alkylierung eines Piperazins der Formel $H-Q^{2B}$ an der Position 1 unter Verwendung eines Alkylierungsmittels der Formel VII

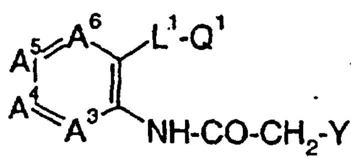

VII

worin Y für eine Abgangsgruppe steht,

(H) für eine Verbindung der Formel I, worin $R^{2A}$ für Methylsulfonyl steht, Substitution des Aminostickstoffs einer entsprechenden Verbindung der Formel I, worin $R^{2A}$ für Wasserstoff steht mittels eines aktivierten Derivats der Methansulfonsäure,

(I) für eine Verbindung der Formel I, worin $R^{2A}$ für $-CHR^yR^z$ oder $-CHR^wR^x$ steht, Alkylierung des Aminostickstoffs einer entsprechenden Verbindung der Formel I, worin $R^{2A}$ für Wasserstoff steht mittels eines Alkylierungsmittels der Formel $Y-CHR^yR^z$ oder $Y-CHR^wR^x$ oder reduktive Alkylierung des Amins mittels einer Verbindung der Formel $R^y-CO-R^z$ oder $R^w-CO-R^x$,

(J) für eine Verbindung der Formel I, worin $R^{2A}$ für 4-Pyridinyl steht (das unsubstituiert ist oder einen Substituenten $R^v$ an der Position 2 oder 3 trägt), Substitution des Aminostickstoffs einer entsprechenden Verbindung der Formel I, worin $R^{2A}$ für Wasserstoff steht mittels eines entsprechenden Pyridinreagenzes, das an der Position 4 eine Abgangsgruppe Y trägt,

(K) für eine Verbindung der Formel 1, worin $R^{2A}$ für 4-Pyridinyl steht, worin $R^v$ für Alkoxycarbonyl steht, Veresterung einer entsprechenden Verbindung der Formel I, worin $R^v$ für Carboxy steht,

(L) für eine Verbindung der Formel I, worin $R^{2A}$ für 4-Pyridinyl steht, worin $R^v$ für Hydroxymethyl steht, Reduktion des Esters einer entsprechenden Verbindung der Formel I, worin $R^v$ für Alkoxycarbonyl steht,

(M) für eine Verbindung der Formel I, worin $R^{2A}$ für 4-Pyridinyl steht, worin $R^v$ für Carbamoyl steht, Amidierung des Esters einer entsprechenden Verbindung der Formel I, worin $R^v$ für Alkoxycarbonyl steht,

(N) für eine Verbindung der Formel I, worin $R^{2A}$ für 4-Pyridinyl steht, worin $R^v$ für Thiocarbamoyl steht, Zugabe von $H_2S$ zum Nitril einer entsprechenden Verbindung der Formel I, worin $R^v$ für Cyano steht,

(O) für eine Verbindung der Formel I, worin $R^{2A}$ für 4-Pyridinyl steht, worin $R^v$ für N-Hydroxyamidino steht, Zugabe von $H_2NOH$ zum Nitril einer entsprechenden Verbindung der Formel I, worin $R^v$ für Cyano steht,

(P) für eine Verbindung der Formel I, worin $R^{2A}$ für 4-Pyridinyl steht, worin $R^v$ für Carboxy steht, Zersetzung des Esters einer entsprechenden Verbindung der Formel I, worin $R^v$ für Alkoxycarbonyl steht,

(Q) für eine Verbindung der Formel I, worin $-NR^sR^t$ nicht für Amino steht, Alkylierung einer entsprechenden Verbindung der Formel I, worin $-NR^sR^t$ für Amino steht, mittels eines herkömmlichen Verfahrens,

(R) für eine Verbindung der Formel I, die $-NR^sR^t$ trägt, reduktive Alkylierung von $H-NR^sR^t$ mittels einer entsprechenden Verbindung, worin aber der Kohlenstoff, der die $-NR^sR^t$ Gruppe tragen muß, eine Oxogruppe trägt,

(S) für eine Verbindung der Formel I, worin $R^p$ für 1-Hydroxy-1-methylethyl steht, Anbindung einer Methylgruppe an die Carbonylgruppe einer entsprechenden Verbindung der Formel I, worin $R^p$ für Acetyl steht, mittels eines Organometallreagenzes,

(T) für eine Verbindung der Formel I, worin $R^p$ für 1-Methoxy-1-methylethyl steht, Behandlung einer entsprechenden Verbindung der Formel I, worin $R^p$ für 1-Hydroxy-1-methylethyl steht, mit Methanol und einem Säurekatalysator, wonach für jedes der obigen Verfahren, wenn eine funktionelle Gruppe mittels einer Schutzgruppe geschützt wird, die Schutzgruppe entfernt wird,
wonach für jedes der obigen Verfahren, wenn ein pharmazeutisch annehmbares Salz einer Verbindung der Formel I erforderlich ist, es durch Umsetzung der basischen Form einer basischen Verbindung der Formel I mit einer Säure, die ein physiologisch annehmbares Gegenion liefert oder der sauren Form einer sauren Verbindung der Formel I mit einer Base, die ein physiologisch annehmbares Gegenion liefert oder durch jedes andere herkömmliche Verfahren erhalten wird,
und worin, falls nichts anderes angegeben ist, $A^3$-$A^6$, $L^1$, $Q^1$ und $R^2$ die in Anspruch 1 definierten Bedeutungen haben.

**10.** Verwendung einer Verbindung der Formel I nach einem der Ansprüche 1 bis 7 zur Herstellung eines Arzneimittels zur Hemmung des Faktors Xa bei einem Säuger.

## Revendications

**1. Composé de formula I**

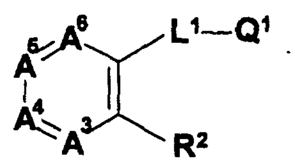

I

(ou un sel de celui-ci acceptable sur le plan pharmaceutique) dans lequel :

$A^3$, $A^4$, $A^5$ et $A^6$, conjointement avec les deux atomes de carbone auxquels ils sont liés, complètent un cycle hétéroaromatique substitué dans lequel

(a) l'un parmi $A^3$, $A^4$, $A^5$ et $A^6$, est N, et chacun des autres est respectivement $CR^3$, $CR^4$, $CR^5$ ou $CR^6$; ou bien

(b) deux résidus non adjacents de $A^3$, $A^4$, $A^5$ et $A^6$, sont chacun N et chacun des autres est respectivement $CR^3$, $CR^4$, $CR^5$ ou $CR^6$; dans lequel

chacun parmi $R^3$, $R^4$, $R^5$ et $R^6$ est indépendamment un atome d'hydrogène ou un groupe méthyle, ou bien l'un parmi $R^3$, $R^4$, $R^5$ et $R^6$, lié à un atome de carbone qui n'est pas lié à un atome de N, est un atome de chlore et les autres sont des atomes d'hydrogène;

$L^1$ est -CO-NH- de telle sorte que -$L^1$-$Q^1$ est -CO-NH-$Q^1$;
$Q^1$ est un groupe 2-pyridinyle (qui porte un substituant méthyle, méthoxy, méthylthio, fluoro ou chloro en la position 5), un groupe 3-pyridinyle (qui porte un substituant méthyle, fluoro ou chloro en la position 6), un groupe 2-pyrimidinyle (qui peut porter un substituant méthyle, fluoro ou chloro en la position 5), un groupe 3-pyridazinyle (qui peut porter un substituant méthyle, fluoro ou chloro en la position 6) ou un groupe 2-benzothiazolyle (qui peut porter un substituant méthyle, fluoro, chloro ou bromo en la position 6);
$R^2$ est -$L^2$-$Q^2$, dans lequel -$L^2$- est -NH-CO-, -NH-CO-X-, -NH-CO-O-X-, -NH-CO-NH-X- ou -NH-$CH_2$-; et $Q^2$ est $Q^{2A}$, $Q^{2B}$, $Q^{2C}$, $Q^{2D}$, $Q^{2E}$ ou $Q^{2F}$, dans lequel X est une liaison simple ou un groupe méthylène, et les valeurs de $L^2$ et $Q^2$ sont choisies ensemble parmi -NH-CO-X-$Q^{2A}$, -NH-CO-O-X-$Q^{2A}$, -NH-CO-NH-X-$Q^{2A}$, -NH-$CH_2$-$Q^{2A}$, -NH-CO-X-$Q^{2B}$, -NH-CO- $Q^{2C}$, -NH-CO-$Q^{2D}$, -NH-CO-$Q^{2E}$, et -NH-CO-$Q^{2F}$ dans lesquels :

$Q^{2A}$ (en indiquant le groupe $L^2$ auquel il est lié) est

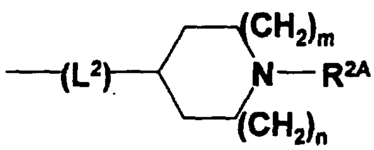

dans laquelle
chacun de m et n vaut indépendamment 0 ou 1, et
$R^{2A}$ est un atome d'hydrogène, un groupe t-butyle, méthylsulfonyle, -$CHR^YR^Z$, -$CHR^WR^X$, ou 4-pyridinyle (qui est non substitué ou porte un substituant $R^V$ en la position 2 ou 3) dans lequel
$R^V$ est un groupe méthyle, hydroxyméthyle, (alcoxy en $C_1$ à $C_2$)carbonyle; cyano, carbamoyle, thiocarbamoyle ou N-hydroxyamidino;
chacun de $R^W$ et $R^X$ est indépendamment un atome d'hydrogène ou un groupe alkyle en $C_1$ à $C_3$ normal; ou -$CHR^WR^X$ est un groupe 2-indanyle ou (en indiquant l'atome d'azote auquel il est lié) est

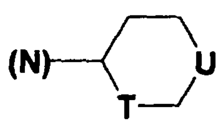

dans laquelle

T est une liaison simple ou un groupe méthylène, et U est un groupe méthylène, éthylène, oxy, -$S(O)_q$- (dans lequel q vaut 0, 1 ou 2) ou imino (qui peut porter un substituant méthyle), ou bien T est un groupe éthane-1,1-diyle et U est une liaison simple ou un groupe méthylène;
$R^Y$ est un atome d'hydrogène ou un groupe méthyle; et
$R^Z$ est un groupe isopropyle, t-butyle, cycloalkyle en $C_3$ à $C_6$, phényle, (qui est non substitué ou porte un ou plusieurs substituants choisis indépendamment parmi un atome d'halogène, un groupe méthyle, méthoxy et hydroxy), 4-quinoléinyle ou hétéroaryle (lequel groupe hétéroaryle est un cycle aromatique à 5 maillons qui comporte un à quatre hétéroatomes choisis parmi l'atome de soufre, d'oxygène et d'azote, ou est un cycle aromatique à 6 maillons qui comporte un à trois atomes d'azote, dans lequel le groupe hétéroaryle est lié à l'atome de carbone, et peut porter un ou plusieurs substituants méthyle sur l'atome de carbone ou d'azote);
$Q^{2B}$ est un groupe 1-pipérazinyle qui porte en la position 4 le groupe $R^{2A}$ (défini ci-dessus);
$Q^{2C}$ est un groupe 3,4-didéshydropipéridin-4-yle qui porte en la position 1 le groupe $R^{2A}$ (défini ci-dessus);

$Q^{2D}$ est un groupe cyclohexyle qui porte en la position 4 le groupe $-NR^SR^T$ dans lequel chacun de $R^S$ et $R^T$ est indépendamment un atome d'hydrogène ou un groupe méthyle, ou bien $R^S$ et $R^T$ ensemble représentent un groupe triméthylène ou tétraméthylène;

$Q^{2E}$ est un groupe 1-pipéridinyle qui porte en la position 4 le groupe $-NR^SR^T$ - (défini ci-dessus); et

$Q^{2F}$ (indiquant le groupe $L^2$ auquel il est lié) est

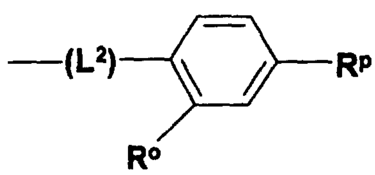

dans laquelle $R^o$ est un atome d'hydrogène, d'halogène, un groupe alkyle en $C_1$ à $C_6$, hydroxy, alcoxy en $C_1$ à $C_4$, benzyloxy, ou alkylthio en $C_1$ à $C_4$; et $R^p$ est un groupe 1-hydroxyéthyle, 1-hydroxy-1-méthyléthyle, 1-méthoxy-1-méthyléthyle, 4-pipéridinyle, 4-pyridinyle, diméthylaminosulfonyle, ou $-J-R^q-$ dans lequel J est une liaison simple, un groupe méthylène, carbonyle, oxy, $-S(O)_q-$ (dans lequel q vaut 0, 1 ou 2), ou $-NR^r-$ (dans lequel $R^r$ est un atome d'hydrogène ou un groupe méthyle); et $R^q$ est un groupe alkyle en en $C_1$ à $C_6$, phényle, 3-pyridyle ou 4-pyridyle.

**2.** Composé selon la revendication 1, dans lequel l'atome d'halogène est un atome de fluor, de chlore, de brome ou d'iode; un groupe alkyle en $C_1$ à $C_2$ est un groupe méthyle ou éthyle; un groupe alkyle en $C_1$ à $C_3$ normal est un groupe méthyle, éthyle ou propyle; un groupe alkyle en $C_1$ à $C_4$ est un groupe méthyle, éthyle, propyle, isopropyle, butyle, isobutyle, ou t-butyle; un groupe alkyle en $C_1$ à $C_6$ est un groupe méthyle, éthyle, propyle; butyle, pentyle ou hexyle; un groupe cycloalkyle en $C_3$ à $C_6$ est un groupe cyclopropyle, cyclobutyle, cyclopentyle ou cyclohexyle.

**3.** Composé selon la revendication 1 ou 2, dans lequel $Q^1$ est un groupe 5-chloropyridin-2-yle ou 6-chloropyridazin-3-yle.

**4.** Composé selon l'une quelconque des revendications 1 à 3, dans lequel $R^2$ est un groupe (1-isopropylpipéridin-4-ylcarbonyl)amino, (1-cyclohexylpipéridin-4-ylcarbonyl)amino, [1-(tétrahydropyran-4-yl)-pipéridin-4-ylcarbonyl] amino, ou [1-(4-pyridinyl)pipéridin-4-ylméthyl]amino.

**5.** Composé selon l'une quelconque des revendications 1 à 4, dans lequel $A^3$ est N et chacun de $A^4$ à $A^6$ est $CR^4$ à $CR^6$ dans lequel chacun de $R^4$ à $R^6$ est un atome d'hydrogène ou bien $R^4$ et $R^6$ sont chacun un atome d'hydrogène et $R^5$ est un atome de chlore.

**6.** Composé selon l'une quelconque des revendications 1 à 4, dans lequel $A^6$ est N et chacun de $A^3$ à $A^5$ est $CR^3$ à $CR^5$ dans lequel chacun de $R^3$ à $R^5$ est un atome d'hydrogène ou bien $R^3$ et $R^4$ sont chacun un atome d'hydrogène et $R^5$ est un groupe méthyle.

**7.** Sel acceptable sur le plan pharmaceutique d'un composé de formula I selon l'une quelconque des revendications 1 à 6, qui est un sel d'addition d'acide fabriqué à partir d'un composé basique de formula I et d'un acide qui fournit un anion acceptable sur le plan pharmaceutique ou bien un sel qui est fabriqué à partir d'un composé acide de formule I et d'une base qui fournit un cation acceptable sur le plan pharmaceutique.

**8.** Formulation pharmaceutique comprenant, en association avec un véhicule, diluant ou excipient, acceptable sur le plan pharmaceutique, un nouveau composé de formula I (ou un sel de celui-ci acceptable sur le plan pharmaceutique) tel que fourni selon l'une quelconque des revendications 1 à 7.

**9.** Procédé de préparation d'un composé de formule 1 (ou un sel de celui-ci acceptable sur le plan pharmaceutique) tel que foumi dans l'une quelconque des descriptions ci-dessus, qui est choisi parmi :

(A) pour un composé de formule I dans lequel $L^2$-$Q^2$ est -NH-CO-$Q^2$, -NH-CO-X-$Q^2$, -NH-CO-O-X-$Q^2$, ou -NH-CO-NH-X-$Q^2$, l'acylation d'une amine de formule II,

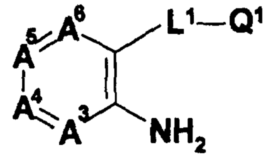

II

en employant un acide correspondant de formule HO-CO-$Q^2$, HO-CO-X-$Q^2$, HO-CO-O-X-$Q^2$, ou HO-CO-NH-X-$Q^2$, ou un dérivé activé de celui-ci;

(B) pour un composé de formule I dans lequel $L^2$-$Q^2$ est -NH-$CH_2$-$Q^2$, la substitution du groupe $Y^a$ d'un composé de formule III

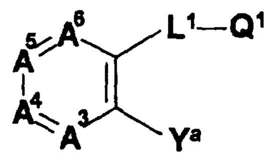

III

dans laquelle $Y^a$ est un groupe sortant classique pour une substitution aromatique nucléophile, par une amine de formule $NH_2$-$CH_2$-$Q^2$;

(C) l'acylation d'une amine de formule $H_2N$-$Q^1$, ou d'un dérivé déprotoné de celle-ci, en employant un acide de formule IV, ou un dérivé activé de celui-ci;

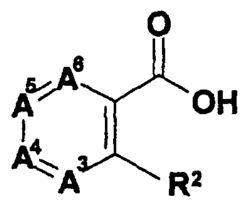

IV

(D) pour un composé de formula I dans lequel $R^2$ est -$NH_2$-$CH_2$-$Q^{2A}$, l'alkylation directe d'une amine de formule II en employant un composé de formula Y-$CH_2$-$Q^{2A}$, ou indirecte par une alkylation réductrice en employant un aldéhyde de formula $Q^{2A}$-CHO;

(E) pour un composé de formula I dans lequel $R^2$ est -NH-CO-O-X-$Q^{2A}$, ou -NH-CO-NH-X-$Q^{2A}$, l'acylation d'un alcool de formule HO-X-$Q^{2A}$ ou d'une amine de formule $NH_2$-X-$Q^{2A}$, en employant un dérivé activé d'un acide de formule VI;

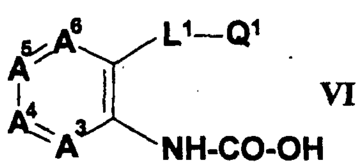

VI

(F) pour un composé de formule I dans lequel $R^2$ est -NH-CO-X-$Q^{2B}$, dans lequel X est une simple liaison, l'acylation au niveau de la position 1 d'une pipérazine de formule H-$Q^{2B}$, en employant un dérivé activé d'un acide de formule VI;

(G) pour un composé de formule I dans lequel $R^2$ est -NH-CO-X-$Q^{2B}$, dans lequel X est un groupe méthylène, l'alkylation au niveau de la position 1 d'une pipérazine de formule H-$Q^{2B}$, en employant un agent d'alkylation de formule VII;

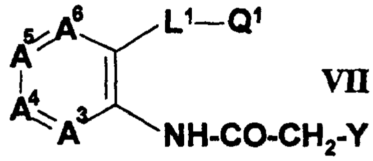

VII

dans laquelle Y est un groupe sortant;

(H) pour un composé de formule I dans lequel $R^{2A}$ est un groupe méthylsulfonyle, la substitution de l'atome d'azote du groupe amino d'un composé correspondant de formule I dans lequel $R^{2A}$ est un atome d'hydrogène en employant un dérivé activé de l'acide méthanesulfonique;

(I) pour un composé de formule I dans lequel $R^{2A}$ est $-CHR^YR^Z$ ou $-CHR^WR^X$, l'alkylation de l'atome d'azote du groupe amino d'un composé correspondant de formule I dans lequel $R^{2A}$ est un atome d'hydrogène en employant un agent d'alkylation de formule $Y-CHR^YR^Z$ ou $Y-CHR^WR^X$ ou l'alkylation réductrice de l'amine en employant un composé de formule $R^Y-CO-R^Z$ ou $R^W-CO-R^X$;

(J) pour un composé de formule I dans lequel $R^{2A}$ est un groupe 4-pyridinyle (qui est non substitué ou porte un substituant $R^V$ en la position 2 ou 3), la substitution de l'atome d'azote du groupe amino d'un composé correspondant de formule I dans lequel $R^{2A}$ est un atome d'hydrogène en employant un réactif pyridine correspondant portant un groupe sortant Y en la position 4;

(K) pour un composé de formule I dans lequel $R^{2A}$ est un groupe 4-pyridinyle dans lequel $R^V$ est un groupe alcoxycarbonyle, l'estérification d'un composé correspondant de formula I dans lequel $R^V$ est un groupe carboxy;

(L) pour un composé de formule I dans lequel $R^{2A}$ est un groupe 4-pyridinyle dans lequel $R^V$ est un groupe hydroxyméthyle, la réduction de l'ester d'un composé correspondant de formule I dans lequel $R^V$ est un groupe alcoxycarbonyle;

(M) pour un composé de formule I dans lequel $R^{2A}$ est un groupe 4-pyridinyle dans lequel $R^V$ est un groupe carbamoyle, l'amidation de l'ester d'un composé correspondant de formule I dans lequel $R^V$ est un groupe alcoxycarbonyle;

(N) pour un composé de formule I dans lequel $R^{2A}$ est un groupe 4-pyridinyle dans lequel $R^V$ est un groupe thiocarbamoyle, l'addition de $N_2S$ au groupe nitrile d'un composé correspondant de formule I dans lequel $R^V$ est un groupe cyano;

(O) pour un composé de formule I dans lequel $R^{2A}$ est un groupe 4-pyridinyle dans lequel $R^V$ est un groupe N-hydroxyamidino, l'addition de $H_2NOH$ au groupe nitrile d'un composé correspondant de formula I dans lequel $R^V$ est un groupe cyano;

(P) pour un composé de formule 1 dans lequel $R^{2A}$ est un groupe 4-pyridinyle dans lequel $R^V$ est un groupe carboxy, la décomposition de l'ester d'un composé correspondant de formule I dans lequel $R^V$ est un groupe alcoxycarbonyle;

(Q) pour un composé de formule I dans lequel $NR^sR^t$ est autre qu'un groupe amino, l'alkylation d'un composé correspondant de formule I dans lequel $-NR^sR^t$ est un groupe amino en employant un procédé classique;

(R) pour un composé de formule I qui porte un groupe $NR^sR^t$, l'alkylation réductrice de $H-NR^sR^t$ en employant un composé correspondant, mais dans lequel l'atome de carbone destiné à porter le groupe $-NR^sR^t$ porte un groupe oxo;

(S) pour un composé de formule I dans lequel $R^p$ est un groupe 1-hydroxy-1-méthyléthyle, l'addition d'un groupe méthyle au groupe carbonyle d'un composé correspondant de formule I dans lequel $R^p$ est un groupe acétyle en employant un réactif organométallique;

(T) pour un composé de formule I dans lequel $R^p$ est un groupe 1-méthoxy-1-méthyléthyle, le traitement d'un composé correspondant de formule I dans lequel $R^p$ est un groupe 1-hydroxy-1-méthyléthyle, par le méthanol et un catalyseur acide;

où par la suite, pour tous les procédés ci-dessus, lorsqu'un groupe fonctionnel est protégé en employant un groupe protecteur, le groupe protecteur est éliminé;

où par la suite, pour l'un quelconque des procédés ci-dessus, lorsqu'on a besoin d'un sel acceptable sur le plan pharmaceutique d'un composé de formule I, on l'obtient en faisant réagir la forme basique d'un composé basique de formule I avec un acide foumissant un contre-ion acceptable sur le plan physiologique ou avec la forme acide d'un composé acide de formule I avec une base fournissant un contre-ion acceptable sur le plan physiologique ou bien à l'aide de tout autre procédé classique;

et dans lequel, sauf indication contraire, $A^3$-$A^6$, $L^1$, $Q^1$ et $R^2$ prennent l'une quelconque des valeurs définies

selon la revendication 1.

**10.** Utilisation d'un composé de formule I selon l'une quelconque des revendications 1 à 7, dans la fabrication d'un médicament pour inhiber le facteur Xa chez un mammifère.